(19) Europäisches Patentamt / European Patent Office / Office européen des brevets

(11) **EP 2 242 744 B1**

(12) **EUROPÄISCHE PATENTSCHRIFT**

(45) Veröffentlichungstag und Bekanntmachung des Hinweises auf die Patenterteilung:
**25.06.2014 Patentblatt 2014/26**

(21) Anmeldenummer: **09700937.7**

(22) Anmeldetag: **08.01.2009**

(51) Int Cl.:
***C07D 233/54*** *(2006.01)* ***C07D 233/56*** *(2006.01)*
***C07D 233/58*** *(2006.01)*

(86) Internationale Anmeldenummer:
**PCT/EP2009/050152**

(87) Internationale Veröffentlichungsnummer:
**WO 2009/087184 (16.07.2009 Gazette 2009/29)**

(54) **Verfahren zur Aufarbeitung ionischer Flüssigkeiten**

Process for working up ionic liquids

Procédé de traitement de liquides ioniques

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **09.01.2008 EP 08150131**

(43) Veröffentlichungstag der Anmeldung:
**27.10.2010 Patentblatt 2010/43**

(73) Patentinhaber: **BASF SE**
**67056 Ludwigshafen (DE)**

(72) Erfinder:
- **BESTE, York Alexander**
  **67056 Ludwigshafen (DE)**
- **STEGMANN, Veit**
  **68167 Mannheim (DE)**
- **MAASE, Matthias**
  **Mendham**
  **New Jersey 07945 (US)**

(74) Vertreter: **Reitstötter Kinzebach**
**Patentanwälte**
**Im Zollhof 1**
**67061 Ludwigshafen (DE)**

(56) Entgegenhaltungen:
**WO-A-2005/021484** **WO-A-2005/070896**
**DE-A1- 10 202 838** **DE-A1-102004 034 543**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 242 744 B1

## Beschreibung

Die vorliegende Erfindung betrifft ein Verfahren zur Aufarbeitung saurer oder basischer ionischer Flüssigkeiten (IL), wie sie beispielsweise aus technischen Verfahren erhalten werden, bei dem man der ionischen Flüssigkeit (IL) ein geeignetes konjugiertes Säure-Base-Paar zusetzt.

Ionische Flüssigkeiten zeichnen sich durch eine Reihe interessanter Eigenschaften aus. Sie sind nicht entzündlich, haben einen äußerst geringen, kaum messbaren Dampfdruck, sind meist umweltfreundlich, verfügen über einen großen Liquidus Bereich und über sehr gute Lösungseigenschaften für zahlreiche Substanzen. Darüber hinaus besitzen ionische Flüssigkeiten aufgrund ihres rein ionischen Aufbaus auch interessante elektrochemische Eigenschaften, wie die elektrische Leitfähigkeit, die häufig von einer hohen elektrochemischen Stabilität begleitet wird. Durch die Auswahl geeigneter Kationen und Anionen lässt sich beispielsweise die Löslichkeit in Wasser oder organischen Lösungsmitteln oder der Schmelzpunkt weitgehend frei bestimmen.

Die molekulare Vielfalt ionischer Flüssigkeiten ermöglicht Ihren Einsatz in einer Vielzahl technischer Anwendungsgebiete. Beispiele hierfür sind die Extraktion (z. B. Gewinnung und Reinigung von technischen Gasen, Isolierung und Reinigung von Kohlenwasserstoffen in der Petrochemie und in der organischen Synthese oder die Entfernung toxischer Substanzen aus Abwässern), die Sorption, Trocknung, Reinigung und Speicherung von Gasen (z. B. in Sorptionsklimaanlagen), die Verwendung als Lösungsmittel (z. B. für die organische Synthese), die Immobilisierung von Katalysatoren, die Verwendung als Schmiermittel, Hydraulikfluid oder Antistatik-Additiv, die Verwendung als Elektrolyt (z. B. bei galvanische Beschichtungen, in Brennstoffzellen, Kondensatoren, Sensor- und Batterietechnik, Metallveredelung, Photovoltaik oder in elektrochromen Bauteilen), die Verwendung als elektroelastischer Werkstoff (z. B. in Aktuatoren), die Verwendung zum Wärmetransport oder zur Wärmespeicherung (z. B. Thermofluide oder PCM-Medien) oder die Verwendung als Spezialanalytikum (z. B. Matrix-Materialien, Lösungsmittel für Karl-Fischer-Titration oder Medien für die Proteinkristallisation oder Elektrophorese).

Häufig ist jedoch der Einsatz ionischer Flüssigkeiten, beispielsweise in den zuvor genannten Anwendungsgebieten, trotz ihrer vorteilhaften Eigenschaften, nicht oder nur für begrenzte Zeit möglich. Ursache hierfür sind im Allgemeinen unerwünschte Wechselwirkungen zwischen der ionischen Flüssigkeit und den damit in Kontakt gebrachten Materialien, wie beispielsweise die Beeinflussung des Verlaufs chemischer Reaktionen oder die Korrosion technischer Anlagen. Solche Wechselwirkungen können in Abhängigkeit der verwendeten ionischen Flüssigkeit und/oder des verwendeten Bereitstellungsverfahrens entweder erst nach einer gewissen Verwendungsdauer oder bereits zu Beginn der Verwendung auftreten und/oder können mit zunehmender Verwendungsdauer in verstärktem Maße auftreten.

Die der vorliegenden Erfindung zugrundeliegende Aufgabe war es, ein Verfahren bereitzustellen, wodurch diejenigen Eigenschaften ionischer Flüssigkeiten, die deren Einsatz begrenzen, verringert werden können. Hierdurch sollten ionische Flüssigkeiten mit denjenigen Eigenschaften ausgestattet oder diejenigen Eigenschaften ionischer Flüssigkeiten wiederhergestellt werden, die für deren vorteilhafte Verwendung notwendig sind. Weiterhin sollten der Einsatz ionischer Flüssigkeiten in Anwendungsgebieten, für die diese vormals ungeeignet waren, und/oder längere Standzeiten ionischer Flüssigkeiten für deren Verwendung in technischen Verfahren und/oder deren Wiederverwertung ermöglicht werden.

Es wurde nun gefunden, dass die Ursache der zuvor genannten störenden Eigenschaften ionischer Flüssigkeiten, häufig die Verunreinigung der ionischen Flüssigkeiten mit geringen Mengen saurer und/oder basischer Verbindungen ist. Diese Verunreinigungen können durch das Bereitstellungsverfahren oder aber beispielsweise durch Zersetzungsreaktionen der ionischen Flüssigkeiten bei Lagerung oder thermischer Belastung bewirkt sein. Derart verunreinigte ionische Flüssigkeiten weisen im Allgemeinen einen sauren oder basischen pH-Wert auf.

Gegenstand der vorliegenden Erfindung ist daher ein Verfahren zur Aufarbeitung einer ionischen Flüssigkeit (IL), ausgewählt unter Verbindungen der allgemeinen Formel (I),

$$[A]^+ \ (1/n)^*[Y]^{n-} \qquad (I)$$

worin $[A]^+$ für ein quartäres Ammonium-Kation und $(1/n)^*[Y]^{n-}$ für ein Anionäquivalent eines n-fach geladenen Anions steht,

wobei die ionische Flüssigkeit (IL) einen sauren oder basischen pH-Wert aufweist,

bei dem man der ionischen Flüssigkeit (IL) ein konjugiertes Säure-Base-Paar, ausgewählt unter Verbindungen der Formel (II) zusetzt,

$$[A]^+ [X]^- \qquad (II)$$

worin $[A]^+$ die für die ionische Flüssigkeit gegebene Bedeutung aufweist und $[X]^-$ für die konjugierte Base steht.

Ionische Flüssigkeiten bezeichnen im Rahmen der vorliegenden Anmeldung organische Salze, die bereits bei Temperaturen unterhalb 180 °C flüssig sind. Vorzugsweise besitzen die ionischen Flüssigkeiten einen Schmelzpunkt von weniger als 180 °C. Weiterhin bevorzugt liegt der Schmelzpunkt in einem Bereich von -50 °C bis 150 °C, besonders bevorzugt im Bereich von -20 °C bis 120 °C und ganz besonders bevorzugt unterhalb 100 °C.

Ionische Flüssigkeiten, die bereits bei Raumtemperatur in flüssigem Aggregatszustand vorliegen, werden beispielsweise von K. N. Marsh et al., Fluid Phase Equilibria 219 (2004), 93 - 98 und J. G. Huddleston et al., Green Chemistry 2001, 3, 156 - 164 beschrieben.

In der ionischen Flüssigkeit liegen Kationen sowie Anionen vor. Dabei kann innerhalb der ionischen Flüssigkeit vom Kation ein Proton oder ein Alkylrest an das Anion übertragen werden, wodurch zwei neutrale Moleküle resultieren. In der erfindungsgemäß eingesetzten ionischen Flüssigkeit kann also ein Gleichgewicht von Anionen, Kationen sowie daraus gebildeten neutralen Molekülen vorliegen.

Im Rahmen der vorliegenden Erfindung umfasst der Ausdruck "Aufarbeitung" die Behandlung ionischer Flüssigkeiten, wie sie aus den Herstellungsverfahren oder durch die Verwendung in technischen Verfahren erhalten werden, mit dem Ziel die ionischen Flüssigkeiten mit denjenigen Eigenschaften auszustatten oder diejenigen Eigenschaften wiederherzustellen, die für die vorteilhafte Verwendung der ionischen Flüssigkeiten benötigt werden. Ziel der Aufarbeitung ist in jedem Fall die Standzeiten ionischer Flüssigkeiten in technischen Verfahren zu erhöhen und/oder deren Wiederverwertung zu ermöglichen.

Der Ausdruck "pH-Wert ionischer Flüssigkeiten" bezeichnet im Rahmen der vorliegenden Erfindung den pH-Wert einer wässrigen Lösung einer ionischen Flüssigkeit und nicht den pH-Wert der ionischen Flüssigkeit an sich. Demzufolge wird der Ausdruck "sauer" im Zusammenhang mit ionischen Flüssigkeiten für solche Verbindungen verwendet, deren wässriger Lösung einen pH-Wert kleiner 7 aufweisen. Der Ausdruck "basisch" wird im Zusammenhang mit ionischen Flüssigkeiten für solche Verbindungen verwendet, deren wässriger Lösung einen pH-Wert größer 7 aufweisen. Zur Bestimmung des "pH-Werts ionischer Flüssigkeiten" wurde jeweils 1 ml der ionischen Flüssigkeit mit Wasser auf ein Gesamtvolumen von 10 ml verdünnt und der pH-Wert der so erhaltenen wässrigen Lösung mit einer Glaselektrode bestimmt.

Im Rahmen der vorliegenden Erfindung bezeichnet der Ausdruck "konjugiertes Säure-Base-Paar" eine Ladungsneutrale Verbindung, die formal aus einem Anion ("konjugierte Base") und einem Kation ("konjugierte Säure") besteht. Aus dem Verhältnis von Basizität der konjugierte Base und Acidität der konjugierte Säure ergeben sich die basischen oder sauren Eigenschaften des konjugierten Säure-Base-Paares, d.h. überwiegt beispielsweise die Basizität der konjugierte Base ist das konjugierte Säure-Base-Paar basisch.

Im Rahmen der vorliegenden Erfindung umfasst der Ausdruck "Alkyl" geradkettiges oder verzweigtes Alkyl. Vorzugsweise handelt es sich um geradkettiges oder verzweigtes $C_1$-$C_{30}$-Alkyl, insbesondere um $C_1$-$C_{18}$-Alkyl und ganz besonders bevorzugt $C_1$-$C_4$-Alkyl. Beispiele für Alkylgruppen sind insbesondere Methyl, Ethyl, n-Propyl, 1-Methylethyl (Isopropyl), n-Butyl, 2-Methylpropyl (Isobutyl), 1-Methylpropyl (sec.-Butyl), 1,1-Dimethylethyl (tert.-Butyl), n-Pentyl, Isopentyl, 1-Methylbutyl, tert.-Pentyl, Neopentyl, n-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, n-Heptyl, n-Octyl, 1-Methylheptyl, 2-Etylhexyl, 2,4,4-Trimethyl-pentyl, 1,1,3,3-Tetramethylbutyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl und n-Eicosyl. Beispiele für $C_1$-$C_4$-Alkyl sind insbesondere Methyl, Ethyl, n-Propyl, Isopropyl, n-Butyl oder tert.-Butyl.

Der Ausdruck Alkyl umfasst auch Alkylreste, deren Kohlenstoffkette durch eine oder mehrere nicht benachbarte Heteroatome oder heteroatomhaltige Gruppen, die vorzugsweise ausgewählt sind unter -O-, -S-, $-NR^E-$, $-PR^E-$, $-SiR^ER^{EE}$ und/oder $-SO_2-$ unterbrochen sein kann. $R^E$ steht vorzugsweise für H, Alkyl, Cycloalkyl, Heterocycloalkyl, Aryl oder Hetaryl. $R^{EE}$ steht vorzugsweise für H, Alkyl, Cycloalkyl, Heterocycloalkyl oder Aryl.

Beispiele für Alkylreste, deren Kohlenstoffketten durch eine oder zwei nicht benachbarte Heteroatome -O- unterbrochen sein können, sind die Folgenden:

Methoxymethyl, Diethoxymethyl, 2-Methoxyethyl, 2-Ethoxyethyl, 2-Propoxyethyl, Diethoxyethyl, 2-Butoxyethyl, 2-Octyloxyethyl, 2-Methoxypropyl, 3-Methoxypropyl, 3-Ethoxypropyl, 3-Propoxypropyl, 2-Isopropoxyethyl, 2-Butoxypropyl, 3-Butoxypropyl, 4-Methoxybutyl, 4-Ethoxybutyl, 4-Propoxybutyl, 6-Methoxyhexyl, 3,6-Dioxa-heptyl (5-Methoxy-3-oxa-pentyl), 3,6-Dioxa-octyl (7-Methoxy-4-oxa-heptyl), 4,8-Dioxa-nonyl (7-Methoxy-4-oxa-heptyl), 3,7-Dioxa-octyl, 3,7-Dioxa-nonyl, 4,7-Dioxa-octyl, 4,7-Dioxanonyl, 2- und 4-Butoxybutyl, 4,8-Dioxadecyl, 9-Ethoxy-5-oxa-nonyl.

Beispiele für Alkylreste, deren Kohlenstoffketten durch drei oder mehr als drei nicht benachbarte Heteroatome

-O- unterbrochen sein können, sind auch Oligo- und Polyoxyalkylene, d. h. Verbindungen mit Wiederholungseinheiten, die vorzugsweise ausgewählt sind unter $(CH_2CH_2O)_{x1}$, $(CH(CH_3)CH_2O)_{x2}$ und $((CH_2)_4O)_{x3}$, wobei x1, x2 und x3 unabhängig voneinander für eine ganze Zahl von 3 bis 100, vorzugsweise 3 bis 80, stehen. Die Summe aus x1, x2 und x3 steht für eine ganze Zahl von 3 bis 300, insbesondere 3 bis 100. In Polyoxyalkylenen, die zwei oder drei verschiedenartige Wiederholungseinheiten aufweisen, ist die Reihenfolge beliebig, d. h. es kann sich um statistisch verteilte, alternierende oder blockförmige Wiederholungseinheiten handeln. Beispiele hierfür sind 3,6,9-Trioxadecyl, 3,6,9-Trioxaundecyl, 3,6,9-Trioxadodecyl, 4,8,12-Trioxatridecyl (11-Methoxy-4,8-dioxa-undecyl), 4,8,12-Trioxatetradecyl, 14-Methoxy-5,10-dioxatetradecyl, 5,10,15-Trioxaheptadecyl, 3,6,9,12-Tetraoxatridecyl, 3,6,9,12-Tetraoxatetradecyl, 4,8,12,16-Tetraoxaheptadecyl (15-Methoxy-4,8,12-trioxa-pentadecyl), 4,8,12,16-Tetraoxa-octadecyl und dergleichen.

Beispiele für Alkylreste, deren Kohlenstoffketten durch eine oder mehrere, z. B. 1, 2, 3, 4 oder mehr als 4, nicht benachbarte Heteroatome -S- unterbrochen sein kann, sind die Folgenden:

Butylthiomethyl, 2-Methylthioethyl, 2-Ethylthioethyl, 2-Propylthioethyl, 2-Butylthioethyl, 2-Dodecylthioethyl, 3-Methylthiopropyl, 3-Ethylthiopropyl, 3-Propylthiopropyl, 3-Butylthiopropyl, 4-Methylthiobutyl, 4-Ethylthiobutyl, 4-Propylthiobutyl, 3,6-Dithiaheptyl, 3,6-Dithia-octyl, 4,8-Dithia-nonyl, 3,7-Dithia-octyl, 3,7-Di-thia-nonyl, 2- und 4-Butylthiobutyl, 4,8-Dithia-decyl, 3,6,9-Trithia-decyl, 3,6,9-Trithia-undecyl, 3,6,9-Trithia-dodecyl, 3,6,9,12-Tetrathia-tridecyl und 3,6,9,12-Tetrathia-tetradecyl.

Beispiele für Alkylreste, deren Kohlenstoffketten durch eine oder zwei nicht benachbarte heteroatomhaltige Gruppen $-NR^E-$ unterbrochen sind, sind die Folgenden:

2-Monomethyl- und 2-Monoethylaminoethyl, 2-Dimethylaminoethyl, 3-Methylaminopropyl, 2- und 3-Dimethylaminopropyl, 3-Monoisopropylaminopropyl, 2- und 4-Monopropylaminobutyl, 2- und 4-Dimethylaminobutyl, 6-Methylaminohexyl, 6-Dimethylaminohexyl, 6-Methyl-3,6-diazaheptyl, 3,6-Dimethyl-3,6-diazaheptyl, 3,6-Diazaoctyl und 3,6-Dimethyl-3,6-diazaoctyl.

Beispiele für Alkylreste, deren Kohlenstoffketten durch drei oder mehr als drei nicht benachbarte heteroatomhaltige Gruppen $-NR^E-$ unterbrochen sein können, sind auch Oligo- und Polyalkylenimine. Das zuvor für die Polyoxyalkylene Gesagte gilt analog für Polyalkylenimine, wobei das Sauerstoffatom jeweils durch eine Gruppe $NR^E$ ersetzt ist, worin $R^a$ vorzugsweise für H oder $C_1$-$C_4$-Alkyl steht. Beispiele hierfür sind 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl, 3,6,9,12-Tetramethyl-3,6,9,12-tetraazatridecyl und dergleichen.

Beispiele für Alkylreste, deren Kohlenstoffketten durch eine oder mehrere, z. B. 1 oder 2 nicht benachbarte Gruppen $-SO_2-$ unterbrochen sind, sind 2-Methylsulfonylethyl, 2-Ethylsulfonylethyl, 2-Propylsulfonylethyl, 2-Isopropylsulfonylethyl, 2-Buthylsulfonylethyl, 2-Methylsulfonylpropyl, 3-Methylsulfonylpropyl, 2-Ethylsulfonylpropyl, 3-Ethylsulfonylpropyl, 2-Propylsulfonylpropyl, 3-Propylsulfonylpropyl, 2-Butylsulfonylpropyl, 3-Butylsulfonylpropyl, 2-Methylsulfonylbutyl, 4-Methylsulfonylbutyl, 2-Ethylsulfonylbutyl, 4-Ethylsulfonylbutyl, 2-Propylsulfonylbutyl, 4-Propylsulfonylbutyl und 4-Butylsulfonylbutyl.

Der Ausdruck Alkyl umfasst auch substituierte Alkylreste. Substituierte Alkylgruppen können in Abhängigkeit von der Länge der Alkylkette einen oder mehrere (z. B. 1, 2, 3, 4, 5 oder mehr als 5) Substituenten aufweisen. Diese sind vorzugsweise unabhängig voneinander ausgewählt unter Cycloalkyl, Cycloalkyloxy, Polycyclyl, Polycyclyloxy, Heterocycloalkyl, Aryl, Aryloxy, Arylthio, Hetaryl, Halogen, Hydroxy, SH, =O, =S, $=NR^E$, COOH, Carboxylat, $SO_3H$, Sulfonat, $NE^1E^2$, Nitro und Cyano, wobei $E^1$ und $E^2$ unabhängig voneinander für H, Alkyl, Cycloalkyl, Heterocycloalkyl, Aryl oder Hetaryl stehen. Cycloalkyl-, Cycloalkyloxy, Polycycloalkyl-, Polycycloalkyloxy-, Heterocycloalkyl-, Aryl- und Hetarylsubstituenten der Alkylgruppen können ihrerseits unsubstituiert oder substituiert sein; geeignete Substituenten sind die nachfolgend für diese Gruppen genannten.

Die vorstehenden Ausführungen zu Alkyl gelten prinzipiell auch für die Alkylteile in Alkoxy, Alkylamino, Dialkylamino, Alkylthio (Alkylsulfanyl), Alkylsulfinyl, Alkylsulfonyl, etc.

Geeignete substituierte Alkylreste sind die Folgenden:

Alkyl, das durch Carboxy substituiert ist, wie z. B. Carboxymethyl, 2-Carboxyethyl, 3-Carboxypropyl, 4-Carboxybutyl, 5-Carboxypentyl, 6-Carboxyhexyl, 7-Carboxyheptyl, 8-Carboxyoctyl, 9-Carboxynonyl, 10-Carboxydecyl, 12-Carboxydodecyl und 14-Carboxytetradecyl;

Alkyl, das durch $SO_3H$ substituiert ist, wie z. B. Sulfomethyl, 2-Sulfoethyl, 3-Sulfopropyl, 4-Sulfobutyl, 5-Sulfopentyl, 6-Sulfohexyl, 7-Sulfoheptyl, 8-Sulfooctyl, 9-Sulfononyl, 10-Sulfodecyl, 12-Sulfododecyl und 14-Sulfotetradecyl;

Alkyl, das durch Carboxylat substituiert ist, wie z. B. für Alkoxycarbonylalkyl, z. B. Methoxycarbonylmethyl, Ethoxycarbonylmethyl, n-Butoxycarbonylmethyl, 2-Methoxycarbonylethyl, 2-Ethoxycarbonylethyl, 2-Methoxycarbonylpropyl, 2-Ethoxycarbonylpropyl, 2-(n-Butoxycarbonyl)propyl, 2-(4-n-Butoxycarbonyl)propyl, 3-Methoxycarbonylpropyl, 3-Ethoxycarbonylpropyl, 3-(n-Butoxycarbonyl)propyl, 3-(4-n-Butoxycarbonyl)propyl, Aminocarbonylalkyl, z. B. Aminocarbonylmethyl, Aminocarbonylethyl, Aminocarbonylpropyl und dergleichen, Alkylaminocarbonylalkyl, wie Methylaminocarbonylmethyl, Methylaminocarbonylethyl, Ethylcarbonylmethyl, Ethylcarbonylethyl und dergleichen, oder Dialkylaminocarbonylalkyl, wie Dimethylaminocarbonylmethyl, Dimethylaminocarbonylethyl, Dimethylcarbonylpropyl, Diethylaminocar-bonylmethyl, Diethylaminocarbonylethyl, Diethylcarbonylpropyl und dergleichen.

Alkyl, das durch Hydroxy substituiert ist, wie z. B. 2-Hydroxyethyl, 2-Hydroxypropyl, 3-Hydroxypropyl, 3-Hydroxybutyl, 4-Hydroxybutyl, 2-Hydroxy-2,2-dimethylethyl, 5-Hydroxy-3-oxa-pentyl, 6-Hydroxyhexyl, 7-Hydroxy-4-oxaheptyl, 8-Hydroxy-4-oxa-octyl, 8-Hydroxy-3,6-dioxa-octyl, 9-Hydroxy-5-oxa-nonyl, 11-Hydroxy-4,8-dioxa-undecyl, 11-Hydroxy-3,6,9-trioxa-undecyl, 14-Hydroxy-5,10-dioxa-tetradecyl, 15-Hydroxy-4,8,12-trioxa-pentadecyl und dergleichen.

Alkyl, das durch Amino substituiert ist, wie z. B. 2-Aminoethyl, 2-Aminopropyl, 3-Aminopropyl, 4-Aminobutyl, 6-Aminohexyl und dergleichen.

Alkyl, das durch Cyano substituiert ist, wie z. B. 2-Cyanoethyl, 3-Cyanopropyl, 3-Cyanobutyl und 4-Cyanobutyl;

Alkyl, das durch Halogen, wie nachfolgend definiert, substituiert ist, wobei in der Alkylgruppe die Wasserstoffatome teilweise oder vollständig durch Halogenatome ersetzt sein können, wie $C_1$-$C_{18}$-Fluoralkyl, z. B. Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl und dergleichen, $C_1$-$C_{18}$-Chloralkyl, z. B. Chlormethyl, Dichlormethyl, Trichlormethyl, 2-Chlorethyl, 2- und 3-Chlorpropyl, 2-, 3- und 4-Chlorbutyl, 1,1-Dimethyl-2-chlorethyl und dergleichen, $C_1$-$C_{18}$-Bromalkyl, z. B. Bromethyl, 2-Bromethyl, 2- und 3-Brompropyl und 2-, 3- und 4-Brombutyl und dergleichen

Alkyl, das durch Nitro substituiert ist, wie z. B. 2-Nitroethyl, 2- und 3-Nitropropyl und 2-, 3- und 4-Nitrobutyl und dergleichen.

Alkyl, das durch Amino substituiert ist, wie z. B. 2-Aminoethyl, 2-Aminopropyl, 3-Aminopropyl, 4-Aminobutyl, 6-Aminohexyl und dergleichen.

Alkyl, das durch Cycloalkyl substituiert ist, wie z. B. Cyclopentylmethyl, 2-Cyclopentylethyl, 3-Cyclopentylpropyl, Cyclohexylmethyl, 2-Cyclohexylethyl, 3-Cyclohexylpropyl und dergleichen.

Alkyl, das durch =O (Oxogruppe) substituiert ist, wie z. B. 2-Oxopropyl, 2-Oxobutyl, 3-Oxobutyl, 1-Methyl-2-oxopropyl, 2-Oxopentyl, 3-Oxopentyl, 1-Methyl-2-oxobutyl, 1-Methyl-3-oxobutyl, 2-Oxohexyl, 3-Oxohexyl, 4-Oxohexyl, 2-Oxoheptyl, 3-Oxoheptyl, 4-Oxoheptyl, 4-Oxoheptyl und dergleichen.

Alkyl, das durch =S (Thioxogruppe) substituiert ist, wie z. B. 2-Thioxopropyl, 2-Thioxobutyl, 3-Thioxobutyl, 1-Methyl-2-thioxopropyl, 2-Thioxopentyl, 3-Thioxopentyl, 1-Methyl-2-thioxobutyl, 1-Methyl-3-thioxobutyl, 2-Thioxohexyl, 3-Thioxohexyl, 4-Thioxohexyl, 2-Thioxoheptyl, 3-Thioxoheptyl, 4-Thioxoheptyl, 4-Thioxoheptyl und dergleichen.

Alkyl, das durch $=NR^E$- substituiert ist, vorzugsweise solches, in denen $R^E$ für H oder $C_1$-$C_4$-Alkyl steht, wie z. B. 2-Iminopropyl, 2-Iminobutyl, 3-Iminobutyl, 1-Methyl-2-iminopropyl, 2-Iminopentyl, 3-Iminopentyl, 1-Methyl-2-iminobutyl, 1-Methyl-3-imino-butyl, 2-Iminohexyl, 3-Iminohexyl, 4-Iminohexyl, 2-Iminoheptyl, 3-Iminoheptyl, 4-Iminoheptyl, 4-Iminoheptyl, 2-Methyliminopropyl, 2-Methyliminobutyl, 3-Methyliminobutyl, 1-Methyl-2-methyliminopropyl, 2-Methyliminopentyl, 3-Methyliminopentyl, 1-Methyl-2-methyliminobutyl, 1-Methyl-3-methyliminobutyl, 2-Methyliminohexyl, 3-Methyliminohexyl, 4-Methyliminohexyl, 2-Methyliminoheptyl, 3-Methyliminoheptyl, 4-Methyliminoheptyl, 4-Methyliminoheptyl, 2-Ethyliminopropyl, 2-Ethyliminobutyl, 3-Ethyliminobutyl, 1-Methyl-2-ethyliminopropyl, 2-Ethyliminopentyl, 3-Ethyliminopentyl, 1-Methyl-2-ethyliminobutyl, 1-Methyl-3-ethyliminobutyl, 2-Ethyliminohexyl, 3-Ethyliminohexyl, 4-Ethyliminohexyl, 2-Ethyliminoheptyl, 3-Ethyliminoheptyl, 4-Ethyliminoheptyl, 4-Ethyliminoheptyl, 2-Propyliminopropyl, 2-Propyliminobutyl, 3-Propyliminobutyl, 1-Methyl-2-propyliminopropyl, 2-Propyliminopentyl, 3-Propyliminopentyl, 1-Methyl-2-propyliminobutyl, 1-Methyl-3-propyliminobutyl, 2-Propyliminohexyl, 3-Propylimino-hexyl, 4-Propyliminohexyl, 2-Propyliminoheptyl, 3-Propyliminoheptyl, 4-Propylimino-heptyl, 4-Propyliminoheptyl und dergleichen.

Alkyl, das durch Aryl substituiert ist ("Arylalkyl"), weist wenigstens eine, wie nachfolgend definierte, unsubstituierte oder substituierte Arylgruppe auf. Geeignete Substituenten an der Arylgruppe sind die nachfolgend genannten. Dabei kann die Alkylgruppe in "Arylalkyl" wenigstens einen weiteren Substituenten, wie vorstehend definiert, tragen und/oder durch eine oder mehrere nicht benachbarte Heteroatome oder hetero-atomhaltige Gruppen, die ausgewählt sind unter -O-, -S-, $-NR^E$-, und/oder $-SO_2$- unterbrochen sein. Arylalkyl steht vorzugsweise für Phenyl-$C_1$-$C_{10}$-alkyl, besonders bevorzugt für Phenyl-$C_1$-$C_4$-alkyl, z. B. für Benzyl, 1-Phenethyl, 2-Phenethyl, 1-Phen-prop-1-yl, 2-Phenprop-1-yl, 3-Phenprop-1-yl, 1-Phenbut-1-yl, 2-Phenbut-1-yl, 3-Phenbut-1-yl, 4-Phenbut-1-yl, 1-Phenbut-2-yl, 2-Phenbut-2-yl, 3-Phenbut-2-yl, 4-Phenbut-2-yl, 1-(Phenmeth)-eth-1-yl, 1-(Phenmethyl)-1-(methyl)-eth-1-yl oder -(Phenmethyl)-1-(methyl)-prop-1-yl; vorzugsweise für Benzyl und 2-Phenethyl.

Alkoxy steht für eine über ein Sauerstoffatom gebundene Alkylgruppe. Beispiele für Alkoxy sind: Methoxy, Ethoxy, n-Propoxy, 1-Methylethoxy, Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methyl-propoxy oder 1-Ethyl-2-methylpropoxy, Hexoxy sowie $R^AO$-$(CH_2CH_2CH_2CH_2O)_n$-$CH_2CH_2CH_2CH_2O$- mit $R^A$ H oder $C_1$-$C_4$-Alkyl, bevorzugt H, Methyl oder Ethyl und n 0 bis 10, bevorzugt 0 bis 3.

Alkylthio (Alkylsulfanyl) steht für eine über ein Schwefelatom gebundene Alkylgruppe. Beispiele für Alkylthio sind Methylthio, Ethylthio, Propylthio, Butylthio, Pentylthio und Hexylthio.

Alkylsulfinyl für eine über eine S(=O)-Gruppe gebundene Alkylgruppe.

Alkylsulfonyl steht für eine über eine $S(=O)_2$-Gruppe gebundene Alkylgruppe.

Der Ausdruck "Alkenyl" umfasst im Sinne der vorliegenden Erfindung geradkettige und verzweigte Alkenylgruppen, die in Abhängigkeit von der Kettenlänge eine oder mehrere Doppelbindungen (z. B. 1, 2, 3, 4 oder mehr als 4) tragen können. Bevorzugt sind $C_2$-$C_{18}$-, besonders bevorzugt $C_2$-$C_{12}$-Alkenylgruppen. Der Ausdruck "Alkenyl" umfasst auch substituierte Alkenylgruppen, welche einen oder mehrere (z. B. 1, 2, 3, 4, 5 oder mehr als 5) Substituenten tragen können. Geeignete Substituenten sind z. B. ausgewählt unter =O, =S, =$NR^E$, Cycloalkyl, Cycloalkyloxy, Polycyclyl, Polycyclyloxy, Hetero-cycloalkyl, Aryl, Aryloxy, Arylthio, Hetaryl, Halogen, Hydroxy, SH, COOH, Carboxylat, $SO_3H$, Sulfonat, Alkylsulfinyl, Alkylsulfonyl, $NE^3E^4$, Nitro und Cyano, wobei $E^3$ und $E^4$ unabhängig voneinander für H, Alkyl, Cycloalkyl, Heterocycloalkyl, Aryl oder Hetaryl stehen.

Der Ausdruck "Alkenyl" umfasst auch Alkenylreste, deren Kohlenstoffkette durch eine oder mehrere nicht benachbarte Heteroatome oder heteroatomhaltige Gruppen, die vorzugsweise ausgewählt sind unter -O-, -S-, -$NR^E$- und/oder -$SO_2$-, unterbrochen sein kann.

Alkenyl steht dann beispielsweise für Ethenyl (Vinyl), 1-Propenyl, 2-Propenyl, 1-Methylethenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl und dergleichen.

Der Ausdruck "Cycloalkyl" umfasst im Rahmen der vorliegenden Erfindung unsubstituierte als auch substituierte monocyclische gesättigte Kohlenwasserstoffgruppen mit im Allgemeinen 3 bis 12 Kohlenstoffringgliedern, vorzugsweise $C_3$-$C_{12}$-Cycloalkylgruppen wie Cyclopropyl, Cyclobutyl, Cyclopentyl, Cyclohexyl, Cycloheptyl, Cyclooctyl, Cyclononyl, Cyclodecyl, Cycloundecyl oder Cyclododecyl, insbesondere $C_5$-$C_{10}$-Cycloalkyl. Geeignete Substituenten sind in der Regel ausgewählt unter für Alkyl, den zuvor für die Alkylgruppen genannten Substituenten, Alkoxy sowie Alkylthio. Substituierte Cycloalkylgruppen können einen oder mehrere (z. B. 1, 2, 3, 4, 5 oder mehr als 5) Substituenten aufweisen, wobei im Falle von Halogen der Cycloalkylrest partiell oder vollständig durch Halogen substituiert ist.

Beispiele für Cycloalkylgruppen sind Cyclopentyl, 2- und 3-Methylcyclopentyl, 2- und 3-Ethylcyclopentyl, Chlorpentyl, Dichlorpentyl, Dimethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Chlorhexyl, Dimethylcyclohexyl, Diethylcyclohexyl, Methoxycyclohexyl, Dimethoxycyclohexyl, Diethoxycyclohexyl, Butoxycyclohexyl, Methylthio-cyclohexyl, Chlorcyclohexyl, Dichlorcyclohexyl, Cycloheptyl, 2-, 3- und 4-Methylcyclo-heptyl, 2-, 3- und 4-Ethylcycloheptyl, 3- und 4-Propylcycloheptyl, 3- und 4-Isopropyl-cycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethyl-cyclooctyl, 3-, 4- und 5-Propylcyclooctyl, partiell fluoriertes Cycloalkyl und perfluoriertes Cycloalkyl der Formel $C_nF_{2(n-a)-(1-b)}H_{2a-b}$ mit n = 5 bis 12, 0 <= a <= n und b = 0 oder 1.

Cycloalkyloxy steht für eine über Sauerstoff gebundene Cycloalkylgruppe, wie vorstehend definiert.

Der Ausdruck "Cycloalkenyl" umfasst unsubstituierte und substituierte, einfach oder zweifach ungesättigte Kohlenwasserstoffgruppen mit 3 bis 5, bis 8, bis 12, vorzugsweise 5 bis 12 Kohlenstoffringgliedern, wie Cyclopent-1-en-1-yl, Cyclopent-2-en-1-yl, Cyclopent-3-en-1-yl, Cyclohex-1-en-1-yl, Cyclohex-2-en-1-yl, Cyclohex-3-en-1-yl, Cyclohexa-2,5-dien-1-yl und dergleichen. Geeignete Substituenten sind die zuvor für Cycloalkyl genannten.

Cycloalkenyloxy steht für eine über Sauerstoff gebundene Cycloalkenylgruppe, wie vorstehend definiert.

Der Ausdruck "Polycyclyl" umfasst im Rahmen der vorliegenden Erfindung im weitesten Sinn Verbindungen, die wenigstens zwei Ringe enthalten, unabhängig davon, wie diese Ringe verknüpft sind. Hierbei kann es sich um carbocyclische und/oder hetero-cyclische Ringe handeln. Die Ringe können gesättigt oder ungesättigt sein. Die Ringe können über Einfach- oder Doppelbindung verknüpft ("mehrkernige Verbindungen"), durch Anellierung verbunden ("kondensierte Ringsysteme") oder überbrückt ("überbrückte Ringsysteme", "Käfigverbindungen") sein. Bevorzugte polycyclische Verbindungen sind überbrückte Ringsysteme und kondensierte Ringsysteme. Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme. Zu den überbrückten Ringsystemen zählen im Rahmen der vorliegenden Erfindung solche, die nicht zu den mehrkernigen Ringsystemen und nicht zu den kondensierten Ringsystemen zählen und bei denen mindestens zwei Ringatome zumindest zwei verschiedenen Ringen angehören. Bei den überbrückten Ringsystemen unterscheidet man je nach Anzahl der Ringöffnungsreaktionen, die formal erforderlich sind, um zu einer offenkettigen Verbindung zu gelangen, Bi-, Tri-,

Tetracyclo- Verbindungen usw., die aus zwei, drei, vier usw. Ringen bestehen. Der Ausdruck "Bicycloalkyl" umfasst dabei bicyclische Kohlenwasserstoffreste mit vorzugsweise 5 bis 10 C-Atomen wie Bicyclo[2.2.1]hept-1-yl, Bicyclo[2.2.1]hept-2-yl, Bicyclo[2.2.1]hept-7-yl, Bicyclo[2.2.2]oct-1-yl, Bicyclo[2.2.2]oct-2-yl, Bicyclo[3.3.0]octyl, Bicyclo[4.4.0]decyl und dergleichen. Der Ausdruck "Bicycloalkenyl" umfasst einfach ungesättigte, bicyclische Kohlenwasserstoffreste mit vorzugsweise 5 bis 10 C-Atomen, wie Bicyclo[2.2.1]hept-2-en-1-yl.

Der Ausdruck "Aryl" umfasst im Rahmen der vorliegenden Erfindung ein- oder mehrkernige aromatische Kohlenwasserstoffreste, die unsubstituiert oder substituiert sein können. Aryl steht in der Regel für Kohlenwasserstoffreste mit 6 bis 10, bis 14, bis 18, vorzugsweise 6 bis 10 Kohlenstoffringgliedern. Aryl steht vorzugsweise für unsubstituiertes oder substituiertes Phenyl, Naphthyl, Anthracenyl, Phenanthrenyl, Naphthacenyl, Chrysenyl, Pyrenyl, etc., und besonders bevorzugt für Phenyl oder Naphthyl. Substituierte Aryle können in Abhängigkeit von der Anzahl und Größe ihrer Ringsysteme einen oder mehrere (z. B. 1, 2, 3, 4, 5 oder mehr als 5) Substituenten aufweisen. Diese sind vorzugsweise unabhängig voneinander ausgewählt unter Alkyl, Alkoxy, Cycloalkyl, Cycloalkyloxy, Heterocycloalkyl, Aryl, Aryloxy, Arylthio, Hetaryl, Halogen, Hydroxy, SH, Alkylthio, Alkylsulfinyl, Alkylsulfonyl, COOH, Carboxylat, $SO_3H$, Sulfonat, $NE^5E^6$, Nitro und Cyano, wobei $E^5$ und $E^6$ unabhängig voneinander für H, Alkyl, Cycloalkyl, Cycloalkyloxy, Polycyclylyl, Polycyclyloxy, Heterocycloalkyl, Aryl, Aryloxy oder Hetaryl stehen. Besonders bevorzugt steht Aryl für Phenyl, das im Falle einer Substitution im Allgemeinen 1, 2, 3, 4 oder 5, vorzugsweise 1, 2 oder 3 Substituenten tragen kann.

Aryl, das einen oder mehrere Reste trägt, steht beispielsweise für 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributyl-phenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl, 2,4,6-Tri-tert.-butylphenyl und 2-, 3-, 4-Dodecylphenyl; 2-, 3- und 4-Methoxyphenyl, 2,4-, 2,5-, 3,5- und 2,6-Dimethoxyphenyl, 2,4,6-Trimethoxy-phenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl, 2-, 3- und 4-Butoxyphenyl, 2-, 3-, 4-Hexyloxyphenyl; 2-, 3-, 4-Chlorphenyl, 2,4-, 2,5-, 3,5- und 2,6-Dichlorphenyl, Trichlorphenyl, 2-, 3-, 4-Fluorphenyl, 2,4-, 2,5-, 3,5- und 2,6-Difluorphenyl, Trifluorphenyl, wie z. B. 2,4,6-Trifluorphenyl, Tetrafluorphenyl, Pentafluorphenyl, 2-, 3- und 4-Cyanophenyl; 2-Nitrophenyl, 4-Nitrophenyl, 2,4-Dinitrophenyl, 2,6-Dinitrophenyl; 4-Dimethylaminophenyl; 4-Acetylphenyl;

Methoxyethylphenyl, Ethoxymethylphenyl; Methylthiophenyl, Isopropylthiophenyl oder tert.-Butylthiophenyl; Methylnaphthyl; Isopropylnaphthyl oder Ethoxynaphthyl. Beispiele für substituiertes Aryl, worin zwei Substituenten, die an benachbarte Kohlenstoffatome des Arylrings gebunden sind, einen kondensierten Ring oder kondensiertes Ringsystem bilden, sind Indenyl und Fluorenyl.

Der Ausdruck "Aryloxy" steht im Rahmen der vorliegenden Erfindung für über ein Sauerstoffatom gebundenes Aryl.

Der Ausdruck "Arylthio" steht im Rahmen der vorliegenden Erfindung für über ein Schwefelatom gebundenes Aryl.

Der Ausdruck "Heterocycloalkyl" umfasst im Rahmen der vorliegenden Erfindung nichtaromatische, ungesättigte oder vollständig gesättigte, cycloaliphatische Gruppen mit im Allgemeinen 5 bis 8 Ringatomen, vorzugsweise 5- oder 6 Ringatomen, in denen 1, 2 oder 3 der Ringkohlenstoffatome durch Heteroatome, ausgewählt unter Sauerstoff, Stickstoff, Schwefel und einer Gruppe $-NR^E-$ ersetzt sind und das unsubstituiert ist oder mit einer oder mehreren, beispielsweise 1, 2, 3, 4, 5 oder 6, $C_1$-$C_6$-Alkylgruppen substituiert ist. Beispielhaft für solche heterocycloaliphatischen Gruppen seien Pyrrolidinyl, Piperidinyl, 2,2,6,6-Tetramethylpiperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothienyl, Dihydrothienyl, Tetrahydrofuranyl, Dihydrofuranyl, Tetrahydropyranyl, 1,2-Oxazolin-5-yl, 1,3-Oxazolin-2-yl und Dioxanyl genannt. Stickstoffhaltiges Heterocycloalkyl kann prinzipiell sowohl über ein Kohlenstoffatom als auch über ein Stickstoffatom gebunden sein.

Der Ausdruck "Heteroaryl (Hetaryl)" umfasst im Rahmen der vorliegenden Erfindung unsubstituierte oder substituierte, heteroaromatische, ein- oder mehrkernige Gruppen, mit im Allgemeine 5 bis 14 Ringatomen, vorzugsweise 5 oder 6 Ringatomen, in denen 1, 2 oder 3 der Ringkohlenstoffatome durch ein, zwei, drei oder vier Heteroatome, ausgewählt unter O, N, $-NR^E-$ und S ersetzt sind, wie Furyl, Thienyl, Oxazolyl, Isoxazolyl, Thiazolyl, Isothiazolyl, Benzofuranyl, Benzthiazolyl, Benzimidazolyl, Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, wobei diese heterocycloaromatischen Gruppen im Falle einer Substitution im Allgemeinen 1, 2 oder 3 Substituenten, tragen können. Die Substituenten sind in der Regel ausgewählt unter $C_1$-$C_6$-Alkyl, $C_1$-$C_6$-Alkoxy, Hydroxy, Carboxy, Halogen und Cyano.

5- bis 7-gliedrige Stickstoff enthaltende Heterocycloalkyl- oder Heteroarylreste, die gegebenenfalls weitere Heteroatome enthalten, stehen beispielsweise für Pyrrolyl, Pyrazolyl, Imidazolyl, Triazolyl, Pyrrolidinyl, Pyrazolinyl, Pyrazolidinyl, Imidazolinyl, Imidazolidinyl, Pyridinyl, Pyridazinyl, Pyrimidinyl, Pyrazinyl, Triazinyl, Piperidinyl, Piperazinyl,

Oxazolyl, Isooxazolyl, Thiazolyl, Isothiazolyl, Indolyl, Chinolinyl, Isochinolinyl oder Chinaldinyl, das unsubstituiert oder substituiert, wie vorstehend genannt sein kann.

Halogen steht für Fluor, Chlor, Brom oder Iod.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäureester- oder Sulfonsäureesterfunktion oder eine Carbonsäure- oder Sulfonsäureamidfunktion. Dazu zählen z. B. die Ester mit $C_1$-$C_4$-Alkanolen, wie Methanol, Ethanol, n-Propanol, Isopropanol, n-Butanol, sec.-Butanol und tert.-Butanol.

Der Ausdruck "Acyl" steht im Sinne der vorliegenden Erfindung für Alkanoyl-, Hetaroyl- oder Aroylgruppen mit im Allgemeinen 1 bis 11, vorzugsweise 2 bis 8 Kohlenstoffatomen, beispielsweise für die Formyl-, Acetyl-, Propanoyl-, Butanoyl-, Pentanoyl-, Hexanoyl-, Heptanoyl-, 2-Ethylhexanoyl-, 2-Propylheptanoyl-, Benzoyl- oder Naphthoyl-Gruppe.

Die Reste $E^1$ und $E^2$, $E^3$ und $E^4$, $E^5$ und $E^6$ sind unabhängig voneinander ausgewählt unter H, Alkyl, Cycloalkyl und Aryl. Die Gruppen $NE^1E^2$, $NE^3E^4$ und $NE^5E^6$ stehen vorzugsweise für N,N-Dimethylamino, N,N-Diethylamino, N,N-Dipropylamino, N,N-Diisopropylamino, N,N-Di-n-butylamino, N,N-Di-tert.-butylamino, N,N-Dicyclohexyl-amino oder N,N-Diphenylamino.

Geeignete Verbindungen, die sich zur Bildung des Kations $[A]^+$ von ionischen Flüssigkeiten eignen, sind z. B. in der DE 102 02 838 A1 beschrieben. Diese Verbindungen enthalten wenigstens ein Stickstoffatom, besonders bevorzugt 1 bis 10 Stickstoffatome, insbesondere 1 bis 5 Stickstoffatome, ganz besonders bevorzugt 1 bis 3 Stickstoffatome und speziell 1 oder 2 Stickstoffatome. Diese Verbindungen können weitere Heteroatome wie Sauerstoff-, Schwefel- oder Phosphoratome enthalten.

Bei der Synthese der ionischen Flüssigkeiten kann zunächst durch Quaternisierung am Stickstoffatom eines Amins oder eines Stickstoff-Heterocyclus ein Ammoniumion erzeugt werden. Die Quaternisierung kann beispielsweise durch Protonierung oder Alkylierung des Stickstoffatoms erfolgen. Je nach verwendetem Protonierungs- oder Alkylierungsmittel werden Salze mit unterschiedlichen Anionen erhalten. In Fällen, in denen es nicht möglich ist, das gewünschte Anion bereits bei der Quaternisierung zu bilden, kann dies in einem weiteren Syntheseschritt erfolgen. Ausgehend beispielsweise von einem Ammoniumhalogenid kann das Halogenid mit einer Lewissäure umgesetzt werden, wobei aus Halogenid und Lewissäure ein komplexes Anion gebildet wird. Alternativ dazu ist der Austausch eines Halogenidions gegen das gewünschte Anion möglich. Dies kann durch Zugabe eines Metallsalzes unter Ausfällung des gebildeten Metallhalogenids, über einen Ionenaustauscher oder durch Verdrängung des Halogenidions durch eine starke Säure (unter Freisetzung der Halogenwasserstoffsäure) geschehen. Geeignete Verfahren sind beispielsweise in Angew. Chem. 2000, 112, S. 3926-3945 und der darin zitierten Literatur beschrieben.

Das zuvor beschriebene Verfahren eignet sich insbesondere zur Aufarbeitung von sauren ionischen Flüssigkeiten, wie sie beispielsweise durch Herstellung aus den entsprechenden Halogeniden, durch thermische Belastung oder teilweise Hydrolyse in technischen Verfahren oder durch Lagerung erhalten werden. Zur Aufarbeitung solcher ionischer Flüssigkeiten eignen sich insbesondere basische konjugierte Säure-Base-Paare, d. h. Verbindungen der Formel (II) die in wässriger Lösung einen pH-Wert von größer 7 aufweisen. Der pH-Wert der Verbindung der Formel (II) wird in wässriger Lösung bestimmt.

Daher werden in einer speziellen Ausführungsform der vorliegenden Erfindung durch das erfindungsgemäße Verfahren ionische Flüssigkeiten (IL) aufgearbeitet, die einen pH-Wert im Bereich von 1 bis 6,9 und insbesondere im Bereich 2 bis 6,5 aufweist bei dem das konjugiertes Säure-Base-Paar der Formel (II) ausgewählt ist unter basischen Verbindungen.

Besonders geeignete basische Säure-Base-Paar der Formel (II) sind solche, worin die konjugierte Base $[X]^-$ für ein Anion der Formel $-OR^1$ steht, worin $R^1$ für Alkyl oder Cycloalkyl steht. Bevorzugt steht $R^1$ für $C_1$-$C_4$-Alkyl und besonders bevorzugt für Methyl, Ethyl, n-Propyl, 1-Methylethyl, n-Butyl, 2-Methylpropyl oder 2,2-Dimethylethyl.

Weiterhin hat es sich als vorteilhaft erwiesen, wenn durch die Neutralisierung der ionischen Flüssigkeit als Neutralisierungsprodukt eine Verbindung der Formel X-H gebildet wird, die sich durch übliche Trennverfahren, insbesondere durch Destillation, von der ionischen Flüssigkeit abtrennen lassen.

In dem erfindungsgemäßen Verfahren ist das Anion $[Y]^{n-}$ der ionischen Flüssigkeiten der Formel (I) bevorzugt ausgewählt unter Verbindungen der Formeln $(Ra)SO_3^-$, $(R^a)SO_2^-$, $(R^a)PO_3^{2-}$, $(R^a)(R^b)PO_2^-$, $(R^a)PO_2^{2-}$, $(R^a)(R^b)PO$-, $(R^a)BO_2^{2-}$, $(R^a)(R^b)BO^-$, $(R^a)(R^b)(R^c)(R^d)B^-$, $(R^a)CO_2^-$, $(R^a)SiO_3^{3-}$, $(R^a)(R^b)SiO_2^{2-}$ und $(R^a)(R^b)(R^c)SiO^-$, worin die Reste $R^a$, $R^b$, $R^c$ und $R^d$ unabhängig voneinander für OH, Alkyl, Alkoxy, Aryl, Aryloxy, Cycloalkyl, Cycloalkoxy, Heterocycloalkyl, Heterocycloalkyloxy, Heteroaryl oder Heteroaryloxy und insbesondere für Alkyl, Alkoxy, Aryl oder Aryloxy stehen.

Ionische Flüssigkeiten, die eines der als bevorzugt genannten Anionen $[Y]^{n-}$ aufweisen, werden im Allgemeinen aus ihren Herstellungsverfahren mit einem pH-Wert von kleiner 7, häufig von 2 bis 6,5, erhalten und/oder unterliegen beispielsweise bei Lagerung oder thermischer Belastung Zersetzungsreaktionen, die zu einer Senkung des pH-Wertes führen. Es wurde nun gefunden, dass diese Eigenschaft der ionischen Flüssigkeiten häufig auf die Verfahren, in denen die ionischen Flüssigkeiten vorteilhafterweise verwendet werden, im Zeitverlauf negativ auswirkt. Hierdurch werden begrenzte Standzeiten der ionischen Flüssigkeiten und somit erhöhte Kosten bewirkt.

Besonders bevorzugt ist $[Y]^{n-}$ in den Verbindungen der Formel (I) ausgewählt unter Verbindungen der Formeln

$(R^a)SO_3^-$, $(R^a)SO_2^-$, $(R^a)PO_3^{2-}$, $(R^a)(R^b)PO_2^-$, $(R^a)PO_2^{2-}$, $(R^a)(R^b)PO^-$ und $(R^a)PO^{2-}$, worin $R^a$ und $R^b$ unabhängig voneinander für OH, Alkyl, Alkoxy, Aryl, Aryloxy, Cycloalkyl, Cycloalkoxy, Heterocycloalkyl, Heterocycloalkyloxy, Heteroaryl oder Heteroaryloxy und insbesondere für Alkyl, Alkoxy, Aryl oder Aryloxy stehen.

Ganz besonders bevorzugt ist $[Y]^{n-}$ in den Verbindungen der Formel (I) ausgewählt unter Verbindungen der Formeln $(R^a)SO_3^-$ und $(R^a)(R^b)PO_2^-$, speziell unter Verbindungen der Formel $(R^a)SO_3^-$, worin $R^a$ und $R^b$ unabhängig voneinander für Alkyl, Alkoxy, Aryl oder Aryloxy stehen.

Bevorzugt stehen $R^a$, $R^b$, $R^c$ und $R^d$ in den Anionen $[Y]^{n-}$ unabhängig voneinander für OH, Alkyl, Alkoxy, Aryl, Aryloxy, Cycloalkyl, Cycloalkoxy, Heterocycloalkyl, Heterocycloalkyloxy, Heteroaryl oder Heteroaryloxy, besonders bevorzugt für Alkyl, Alkoxy, Aryl oder Aryloxy und ganz besonders bevorzugt für $C_1$-$C_4$-Alkyl, $C_1$-$C_4$-Alkoxy oder Phenyl, wobei Phenyl unsubstituiert ist oder 1, 2 oder 3 Substituenten ausgewählt unter Halogen, CN, $NO_2$, $C_1$-$C_4$-Alkyl, $C_1$-$C_4$-Alkoxy und Di($C_1$-$C_4$-alkyl)amino trägt.

Bevorzugte Kationen $[A]^+$ sind solche Verbindungen, die eine molare Masse von weniger als 1000 g/mol aufweisen, ganz besonders bevorzugt von weniger als 600 g/mol und insbesondere von weniger 400 g/mol.

Bevorzugte Kationen $[A]^+$ sind weiterhin solche Verbindungen, die mindestens einen fünf- bis sechsgliedrigen Heterocyclus, insbesondere einen fünfgliedrigen Heterocyclus, enthalten, der mindestens ein Stickstoffatom sowie gegebenenfalls ein Sauerstoff- oder Schwefelatom aufweist, besonders bevorzugt sind solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit zwei Stickstoffatomen. Weiterhin bevorzugt sind aromatische Heterocyclen.

Demzufolge wird man in einer bevorzugten Ausführungsform des erfindungsgemäßen Verfahrens eine ionische Flüssigkeit (IL) aufarbeiten, die ein heterocyclisches Kation $[A]^+$ aufweist.

Im Rahmen der vorliegenden Erfindung umfasst Der Begriff "heterocyclisches" Kation sowohl "heteroaromatische" Kationen als auch "teilweise oder vollständig gesättigte heterocyclische Kationen".

Der Begriff "heteroaromatisches" Kation umfasst Kationen, deren Struktur sich beispielsweise durch Quaternisierung eines Ringstickstoffatoms einer "Hetaryl"-Verbindung, wie zuvor definiert, herleiten lässt. Beispiele fünf- oder sechsgliedriger heteroaromatischer Kationen sind Pyrazolium, Oxazolium, Isoxazolium, Thiazolium, Isothiazolium, Imidazolium, 1,2,4-Oxadiazolium, 1,2,4-Thiadiazolium, 1,3,4-Oxadiazolium, 1,3,4-Thiadiazolium, Pyrrolium, 1,2,3-Triazolium, 1,2,4-Triazolium, Pyridinium, Pyridazinium, Pyrimidinium, 2-Pyrazinium, 1,3,5-Triazinium und 1,2,4-Triazinium.

Der Begriff "teilweise oder vollständig gesättigtes" heterocyclisches Kation umfasst Kationen, deren Struktur sich beispielsweise durch Quaternisierung eines Ringstickstoffatoms einer "Heterocycloalkyl"-Verbindung, wie zuvor definiert, herleiten lässt. Beispiele fünf- oder sechsgliedriger gesättigter oder partiell ungesättigter heterocyclischer Kationen sind Pyrrolidinium, Pyrazolidinium, Oxazolidinium, Isoxazolidinium, Thiazolidinium, Isothiazolidinium, Imidazolidinium, 1,2,4-Oxadiazolidinium, 1,2,4-Thiadiazolidinium, 1,2,4-Triazolidinium, 1,3,4-Oxadiazolidinium, 1,3,4-Thiadiazolidinium, 1,3,4-Triazolidinium, 2-Pyrrolinium, 3-Pyrrolinium, 2-Isoxazolinium, 3-Isoxazolinium, 4-Isoxazolinium, 2-Isothiazolinium, 3-Isothiazolinium, 4-Isothiazolinium, 2,3-Dihydropyrazolium, 3,4-Dihydropyrazolium, 4,5-Dihydropyrazolium, 2,3-Dihydrooxazolium, 3,4-Dihydrooxazolium, Piperidinium, Hexahydropyridazinium, Hexahydropyrimidinium, Piperazinium, 1,3,5-Hexahydrotriazinium oder 1,2,4-Hexahydrotriazinium.

Bevorzugt weist die erfindungsgemäß aufgearbeitete ionische Flüssigkeit (IL) wenigstens ein Kation auf, das ausgewählt ist unter den im Folgenden gezeigten Verbindungen der Formeln (IV.a) bis (IV.v),

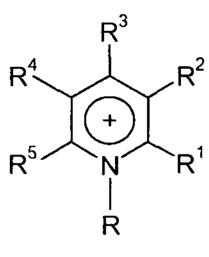

(IV.a)

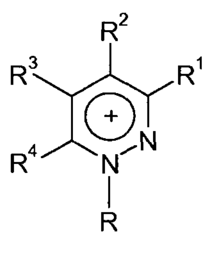

(IV.b)

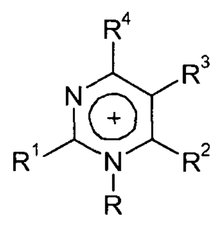

(IV.c)

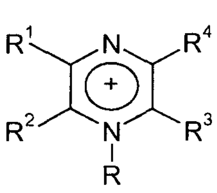

(IV.d)

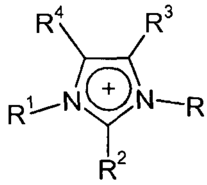

(IV.e)

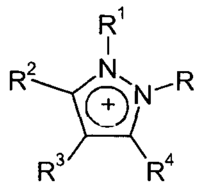

(IV.f)

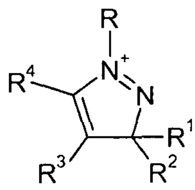

(IV.g)

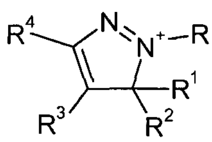

(IV.g')

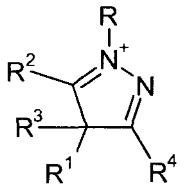

(IV.h)

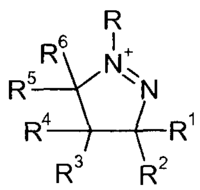

(IV.i)

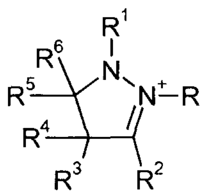

(IV.j)

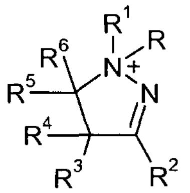

(IV.j')

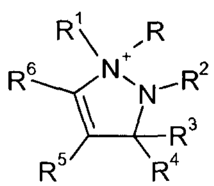

(IV.k)

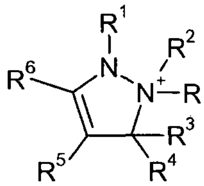

(IV.k')

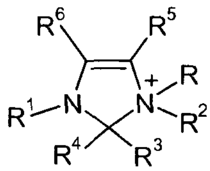

(IV.l)

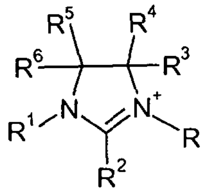

(IV.m)

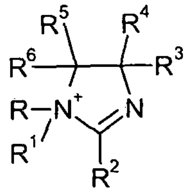

(IV.m')

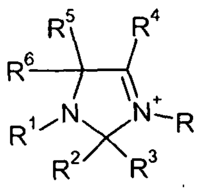

(IV.n)

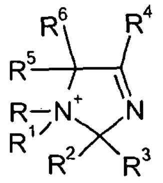

(IV.n')

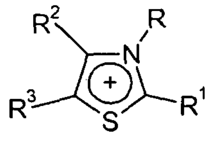

(IV.o)

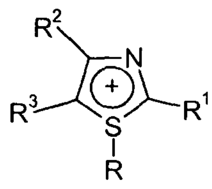

(IV.o')

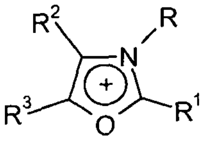

(IV.p)

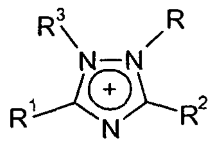

(IV.q)

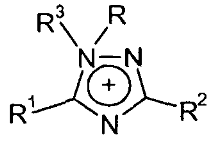

(IV.q')

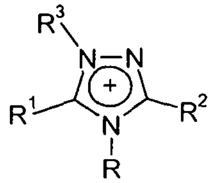

(IV.q")

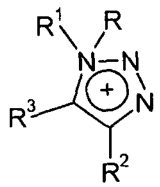

(IV.r)

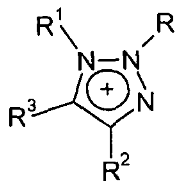

(IV.r')

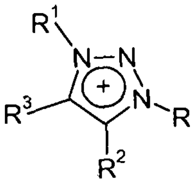

(IV.r")

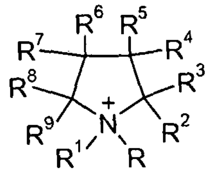

(IV.s)

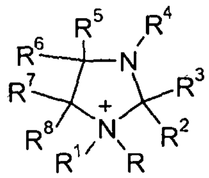

(IV.t)

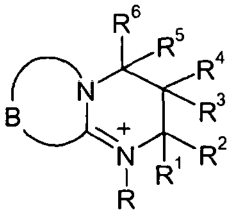

(IV.u)

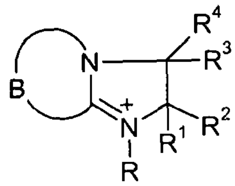

(IV.v)

sowie Oligomeren, die diese Strukturen enthalten, worin
R für H, Alkyl, Alkenyl, Cycloalkyl, Cycloalkenyl, Polycyclyl, Heterocycloalkyl, Aryl oder Heteroaryl stehen;
Reste $R^1$, $R^2$, $R^3$, $R^4$, $R^5$, $R^6$, $R^7$, $R^8$ und $R^9$, die an ein Ringkohlenstoffatom gebunden sind, unabhängig voneinander für H, eine Sulfogruppe, COOH, Carboxylat, Sulfonat, Acyl, Alkoxycarbonyl, Cyano, Halogen, Hydroxyl, SH, Nitro, $NE^1E^2$, Alkyl, Alkoxy, Alkylthio, Alkylsulfinyl, Alkylsulfonyl, Alkenyl, Cycloalkyl, Cycloalkyloxy, Cycloalkenyl, Cycloalkenyloxy, Polycyclyl, Polycyclyloxy, Heterocycloalkyl, Aryl, Aryloxy oder Heteroaryl stehen, wobei $E^1$ und $E^2$ unabhängig voneinander für H, Alkyl, Cycloalkyl, Heterocycloalkyl, Aryl oder Hetaryl stehen,
Reste $R^1$, $R^2$, $R^3$, $R^4$, $R^5$, $R^6$, $R^7$, $R^8$ und $R^9$, die an ein Ringheteroatom gebunden sind, für H, $SO_3H$, $NE^1E^2$, Alkyl, Alkoxy, Alkenyl, Cycloalkyl, Cycloalkenyl, Polycyclyl, Heterocycloalkyl, Aryl oder Heteroaryl, stehen, wobei $E^1$ und $E^2$

unabhängig voneinander für H, Alkyl, Cycloalkyl, Heterocycloalkyl, Aryl oder Hetaryl stehen, oder
zwei benachbarte Reste $R^1$ bis $R^9$ auch zusammen mit den Ringatomen, an die sie gebunden sind, für wenigstens einen anellierten, gesättigten, ungesättigten oder aromatischen Ring oder ein Ringsystem mit 1 bis 30 Kohlenstoffatomen stehen können, wobei der Ring oder das Ringsystem 1 bis 5 nicht benachbarte Heteroatome oder heteroatomhaltige Gruppen aufweisen kann und wobei der Ring oder das Ringsystem unsubstituiert oder substituiert sein kann,
wobei zwei geminale Reste $R^1$ bis $R^9$ auch gemeinsam für =O, =S oder =$NR^b$ stehen können, wobei $R^b$ für H, Alkyl, Cycloalkyl, Aryl oder Heteroaryl steht,
wobei in den Verbindungen der Formel (IV.u) $R^1$ und $R^3$ oder $R^3$ und $R^5$ auch gemeinsam für den Bindungsanteil einer Doppelbindung zwischen den Ringatomen, die diese Reste tragen, stehen können,
B in den Verbindungen der Formeln (IV.u) und (IV.v) zusammen mit der CN-Gruppe, an die es gebunden ist, einen 4- bis 8-gliedrigen, gesättigten oder ungesättigten oder aromatischen Cyclus bildet, der gegebenenfalls substituiert ist und/oder der gegebenenfalls weitere Heteroatome oder heteroatomhaltige Gruppen aufweisen kann und/oder der weitere anellierte gesättigte, ungesättigte oder aromatische Carbocyclen oder Heterocyclen umfassen kann.

Bezüglich der allgemeinen Bedeutung der zuvor genannten Reste Carboxylat, Sulfonat, Acyl, Alkoxycarbonyl, Halogen, $NE^1E^2$, Alkyl, Alkoxy, Alkylthio, Alkylsulfinyl, Alkyl-sulfonyl, Alkenyl, Cycloalkyl, Cycloalkyloxy, Cycloalkenyl, Cycloalkenyloxy, Polycyclyl, Polycyclyloxy, Heterocycloalkyl, Aryl, Aryloxy oder Heteroaryl wird auf die eingangs gemachten Ausführungen im vollen Umfang Bezug genommen. Reste $R^1$ bis $R^9$, welche in den oben genannten Formeln (IV) an ein Kohlenstoffatom gebunden sind und ein Heteroatom oder eine heteroatomhaltige Gruppe aufweisen, können auch direkt über ein Heteroatom an das Kohlenstoffatom gebunden sein.

Bilden zwei benachbarte Reste $R^1$ bis $R^9$ zusammen mit den Ringatomen, an die sie gebunden sind, wenigstens einen anellierten, gesättigten, ungesättigten oder aromatischen Ring oder ein Ringsystem mit 1 bis 30 Kohlenstoffatomen, wobei der Ring oder das Ringsystem 1 bis 5 nicht benachbarte Heteroatome oder heteroatomhaltige Gruppen aufweisen kann und wobei der Ring oder das Ringsystem unsubstituiert oder substituiert sein kann, so können diese Reste gemeinsam als anellierte Bausteine vorzugsweise 1,3-Propylen, 1,4-Butylen, 1,5-Pentylen, 2-Oxa-1,3-propylen, 1-Oxa-1,3-propylen, 2-Oxa-1,3-propylen, 1-Oxa-1,3-propenylen, 3-Oxa-1,5-pentylen, 1-Aza-1,3-propenylen, 1-$C_1$-$C_4$-Alkyl-1-aza-1,3-propenylen, 1,4-Buta-1,3-dienylen, 1-Aza-1,4-buta-1,3-dienylen oder 2-Aza-1,4-buta-1,3-dienylen bedeuten.

Bevorzugt steht der Rest R in den Verbindungen der Formeln IV.a bis IV.v für
unsubstituiertes $C_1$-$C_{18}$-Alkyl wie Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl und 1-Octadecyl;
ein- bis mehrfach mit Hydroxy, Halogen, Phenyl, Cyano, $C_1$-$C_6$-Alkoxycarbonyl und/oder $SO_3H$ substituiertes $C_1$-$C_{18}$-Alkyl, speziell Hydroxy-$C_1$-$C_{18}$-alkyl, wie z. B. 2-Hydroxyethyl oder 6-Hydroxyhexyl; Phenyl-$C_1$-$C_{18}$-alkyl, wie z. B. Benzyl, 3-Phenylpropyl; Cyano-$C_1$-$C_{18}$-alkyl, wie z. B. 2-Cyanoethyl; $C_1$-$C_6$-Alkoxy-$C_1$-$C_{18}$-alkyl, wie z. B. 2-(Methoxycarbonyl)-ethyl, 2-(Ethoxycarbonyl)-ethyl oder 2-(n-Butoxy-carbonyl)-ethyl; $C_1$-$C_{18}$-Fluoralkyl, wie Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl; Sulfo-$C_1$-$C_{18}$-alkyl, wie z. B. 3-Sulfopropyl;
Hydroxyethyloxyalkyl, Reste von Oligo- und Polyalkylenglycolen wie Polyethylenglycole und Polypropylenglycolen und deren Oligomere mit 2 bis 100 Einheiten und einem H oder einem $C_1$-$C_8$-Alkyl als Endgruppe, wie beispielsweise $R^AO$-$(CHR^B$-$CH_2$-$O)_n$-$CHR^B$-$CH_2$- mit $R^A$ und $R^B$ bevorzugt H, Methyl oder Ethyl und n bevorzugt 0 bis 3, insbesondere 3-Oxa-butyl, 3-Oxa-pentyl, 3,6-Dioxa-heptyl, 3,6-Dioxa-octyl, 3,6,9-Trioxa-decyl, 3,6,9-Trioxa-undecyl, 3,6,9,12-Tetraoxa-tridecyl und 3,6,9,12-Tetraoxa-tetradecyl; und
$C_2$-$C_6$-Alkenyl, wie Vinyl oder Propenyl.

Besonders bevorzugt steht der Rest R für lineares $C_1$-$C_{18}$-Alkyl, wie beispielsweise Methyl, Ethyl, 1-Propyl, 1-Butyl, 1-Pentyl, 1-Hexyl, 1-Heptyl, 1-Octyl, 1-Decyl, 1-Dodecyl, 1-Tetradecyl, 1-Hexadecyl, 1-Octadecyl, ganz besonders bevorzugt für Methyl, Ethyl, 1-Butyl und 1-Octyl sowie für $CH_3O$-$(CH_2CH_2O)_n$-$CH_2CH_2$- und $CH_3CH_2O$-$(CH_2CH_2O)_m$-$CH_2CH_2$-, worin m für 0 bis 3 steht.

Bevorzugt stehen die Reste $R^1$ bis $R^9$ in den Verbindungen der Formel IV.a bis IV.v unabhängig voneinander für H, Halogen, Hydroxy, Alkoxy, Alkylthio, Carboxyl, -COOH, Sulfonat, CN, $NO_2$, Acyl, Alkoxycarbonyl, $NE^1E^2$, worin $E^1$ und $E^2$ eine der zuvor gegebenen Bedeutungen aufweisen,
$C_1$-$C_{18}$-Alkyl, das unsubstituiert oder substituiert ist und/oder durch wenigstens ein Heteroatom oder eine heteroatomhaltige Gruppe unterbrochen sein kann, $C_2$-$C_{18}$-Alkenyl, das unsubstituiert oder substituiert ist und/oder durch wenigstens ein Heteroatom unterbrochen sein kann,
$C_6$-$C_{10}$-Aryl, das unsubstituiert oder substituiert ist,
$C_5$-$C_{12}$-Cycloalkyl, das unsubstituiert oder substituiert ist,
Polycyclyl, das unsubstituiert oder substituiert ist,

$C_5$-$C_{12}$-Cycloalkenyl, das unsubstituiert oder substituiert ist,

Heterocycloalkyl mit 5 oder 6 Ringatomen, wobei der Ring neben Kohlenstoffringgliedern 1, 2 oder 3 Heteroatome oder heteroatomhaltige Gruppen aufweist, die ausgewählt sind unter Sauerstoff, Stickstoff, Schwefel und $NR^E$, und das unsubstituiert oder substituiert ist oder

Heteroaryl mit 5 bis 10 Ringatomen, wobei der Ring neben Kohlenstoffringgliedern 1, 2 oder 3 Heteroatome oder heteroatomhaltige Gruppen aufweist, die ausgewählt sind unter Sauerstoff, Stickstoff, Schwefel und $NR^E$, und das unsubstituiert oder substituiert ist.

Ebenfalls bevorzugt stehen in den Verbindungen der Formeln IV.a bis IV.v zwei benachbarte Reste $R^1$ bis $R^9$ zusammen mit den Ringatomen, an die sie gebunden sind, für wenigstens einen anellierten, gesättigten, ungesättigten oder aromatischen Ring oder für ein Ringsystem mit 1 bis 12 Kohlenstoffatomen, wobei der Ring oder das Ringsystem 1 bis 5 nicht benachbarte Heteroatome oder heteroatomhaltige Gruppen aufweisen kann, die vorzugsweise ausgewählt sind unter Sauerstoff, Stickstoff, Schwefel und $NR^E$, und wobei der Ring oder das Ringsystem unsubstituiert ist oder substituiert sein kann, wobei die Substituenten vorzugsweise unabhängig voneinander ausgewählt sind unter Alkoxy, Cycloalkyl, Cycloalkoxy, Polycyclyl, Polycyclyloxy, Heterocycloalkyl, Aryl, Aryloxy, Arylthio, Heteroaryl Halogen, Hydroxy, SH, =O, =S, $=NR^E$, COOH, Carboxylat, $-SO_3H$, Sulfonat, $NE^1E^2$, Nitro und Cyano, wobei $E^1$ und $E^2$ unabhängig voneinander für H, Alkyl, Cycloalkyl, Heterocycloalkyl, Aryl oder Hetaryl stehen.

Wenn $R^1$ bis $R^9$ in den Verbindungen der Formel IV.a bis IV.v für Alkoxy stehen, so stehen $R^1$ bis $R^9$ vorzugsweise für Methoxy oder Ethoxy oder für $R^AO-(CH_2CH_2CH_2CH_2O)_n-CH_2CH_2CH_2CH_2O-$, worin $R^A$ und $R^B$ bevorzugt für H, Methyl oder Ethyl stehen und n bevorzugt für 0 bis 3 steht.

Wenn $R^1$ bis $R^9$ in den Verbindungen der Formel IV.a bis IV.v für Acyl stehen, sind diese vorzugsweise ausgewählt unter Formyl und $C_1$-$C_4$-Alkylcarbonyl, insbesondere unter Formyl oder Acetyl.

Wenn $R^1$ bis $R^9$ in den Verbindungen der Formel IV.a bis IV.v für $C_1$-$C_{18}$-Alkyl stehen, sind diese vorzugsweise ausgewählt unter unsubstituiertem $C_1$-$C_{18}$-Alkyl, wie Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-9-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, 2-Etylhexyl, 2,4,4-Trimethyl-pentyl, 1,1,3,3-Tetramethylbutyl, 1-Nonyl, 1-Decyl, 1-Undecyl, 1-Dodecyl, 1-Tridecyl, 1-Tetradecyl, 1-Pentadecyl, 1-Hexadecyl, 1-Heptadecyl, 1-Octadecyl; $C_1$-$C_{18}$-Halogenalkyl, speziell $C_1$-$C_{18}$-Fluoralkyl, beispielsweise Trifluormethyl, Difluormethyl, Fluormethyl, Pentafluorethyl, Heptafluorpropyl, Heptafluorisopropyl, Nonafluorbutyl, Nonofluorisobutyl, Undecylfluorpentyl, Undecylisopentyl, $C_6F_{13}$, $C_8F_{17}$, $C_{10}F_{21}$, $C_{12}F_{25}$, speziell $C_1$-$C_{18}$-Chloralkyl, wie Chlormethyl, 2-Chlorethyl, Trichlormethyl, 1,1-Dimethyl-2-chlorethyl;

Amino-$C_1$-$C_{18}$-alkyl, wie 2-Aminoethyl, 2-Aminopropyl, 3-Aminopropyl, 4-Aminobutyl, 6-Aminohexyl,

$C_1$-$C_6$-Alkylamino-$C_1$-$C_{18}$-alkyl, wie 2-Methylaminoethyl, 2-Methylaminopropyl, 3-Methylaminopropyl, 4-Methylaminobutyl, 6-Methylaminohexyl; Di-($C_1$-$C_6$-alkyl)-$C_1$-$C_{18}$-alkyl, wie 2-Dimethylaminoethyl, 2-Dimethylaminopropyl, 3-Dimethylaminopropyl, 4-Dimethylaminobutyl, 6-Dimethylaminohexyl, Cyano-$C_1$-$C_{18}$-alkyl, wie 2-Cyanoethyl, 2-Cyanopropyl, $C_1$-$C_{10}$-Alkoxy-$C_1$-$C_{18}$-alkyl, wie Methoxymethyl, 2-Methoxyethyl, 2-Methoxypropyl, 3-Methoxypropyl, 2-Methoxyisopropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, 2-Isopropoxyethyl, 2-Butoxyethyl, 2-Butoxypropyl, 2-Octyloxyethyl, 5-Methoxy-3-oxa-pentyl, 8-Methoxy-3,6-dioxa-octyl, 7-Methoxy-4-oxa-heptyl, 11-Methoxy-4,8-dioxa-undecyl, 9-Methoxy-5-oxa-nonyl, 9-Methoxy-5-oxa-nonyl, 14-Methoxy-5,10-dioxa-tetradecyl, 5-Ethoxy-3-oxa-pentyl, 8-Ethoxy-3,6-dioxa-octyl, 7-Ethoxy-4-oxa-heptyl, 11-Ethoxy-4,8-dioxa-undecyl, 9-Ethoxy-5-oxa-nonyl oder 14-Ethoxy-5,10-oxa-tetradecyl, 15-Methoxy-4,8,12-trioxa-pentadecyl, 11-Methoxy-3,6,9-trioxa-undecyl, 11-Ethoxy-3,6,9-trioxa-undecyl, 15-Ethoxy-4,8,12-trioxa-pentadecyl; Di-($C_1$-$C_{10}$-alkoxy-$C_1$-$C_{18}$-alkyl), wie Diethoxymethyl oder Diethoxyethyl, $C_1$-$C_6$-Alkoxycarbonyl-$C_1$-$C_{18}$-alkyl, wie 2-(Methoxycarbonyl)-ethyl, 2-(Ethoxycarbonyl)-ethyl, 2-(n-Butoxycarbonyl)-ethyl, Di-($C_1$-$C_6$-alkoxycarbonyl)-$C_1$-$C_{18}$-alkyl, wie 1,2-Di-(methoxycarbonyl)-ethyl, Hydroxy-$C_1$-$C_{18}$-alkyl, wie 2-Hydroxyethyl, 2-Hydroxypropyl, 3-Hydroxypropyl, 4-Hydroxybutyl, 6-Hydroxyhexyl, 2-Hydroxy-2,2-dimethylethyl, 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-dioxa-tetradecyl; $C_1$-$C_{12}$-Alkylsulfanyl-$C_1$-$C_{18}$-alkyl, wie Butylthiomethyl, 2-Dodecylthioethyl, $C_5$-$C_{12}$-Cycloalkyl-$C_1$-$C_{18}$-alkyl, wie Cyclopentylmethyl, 2-Cyclopentylethyl, 3-Cyclopentylpropyl, Cyclohexylmethyl, 2-Cyclohexylethyl, 3-Cyclohexylpropyl, Phenyl-$C_1$-$C_{18}$-alkyl, wobei der Phenylteil von Phenyl-$C_1$-$C_{18}$-alkyl unsubstituiert ist oder ein-, zwei-, drei- oder vierfach substituiert ist und die Substituenten unabhängig voneinander unter $C_1$-$C_{18}$-Alkyl, Halogen, $C_1$-$C_6$-Alkoxy und Nitro ausgewählt sind, wie Benzyl (Phenylmethyl), 1-Phenylethyl, 2-Phenylethyl, 3-Phenylpropyl, p-Tolylmethyl, 1-(p-Butylphenyl)-ethyl, p-Chlorbenzyl, 2,4-Dichlorbenzyl, p-Methoxybenzyl, m-Ethoxybenzyl, Phenyl-$C(CH_3)_2$-, 2,6-Dimethylphenylmethyl, Diphenyl-$C_1$-$C_{18}$-alkyl, wie Diphenylmethyl (Benzhydryl); Triphenyl-$C_1$-$C_{18}$-alkyl, wie Triphenylmethyl; Phenoxy-$C_1$-$C_{18}$-alkyl, wie 2-Phenoxyethyl, 2-Phenoxypropyl, 3-Phenoxypropyl, 4-Phenoxybutyl, 6-Phenoxyhexyl; und Phenylthio-$C_1$-$C_{18}$-alkyl, wie 2-Phenylthioethyl.

Wenn $R^1$ bis $R^9$ in den Verbindungen der Formel IV.a bis IV.v für $C_2$-$C_{18}$-Alkenyl stehen, sind diese vorzugsweise

ausgewählt unter $C_2$-$C_6$-Alkenyl, wie Vinyl, 2-Propenyl, 3-Butenyl, cis-2-Butenyl, trans-2-Butenyl oder $C_2$-$C_{18}$-Alkenyl, das teilweise oder vollständig durch Fluor substituiert ist.

Wenn $R^1$ bis $R^9$ in den Verbindungen der Formel IV.a bis IV.v für $C_6$-$C_{10}$-Aryl stehen, so stehen $R^1$ bis $R^9$ vorzugsweise für Phenyl oder Naphthyl, wobei Phenyl oder Naphthyl unsubstituiert ist oder ein-, zwei-, drei- oder vierfach substituiert ist, wobei die Substituenten unabhängig voneinander unter Halogen, $C_1$-$C_{15}$-Alkyl, $C_1$-$C_6$-Alkoxy, $C_1$-$C_6$-Alkylsulfanyl, $C_1$-$C_6$-Alkoxy-$C_1$-$C_6$-alkyl, $C_1$-$C_6$-Alkylcarbonyl, Amino, $C_1$-$C_6$-Alkylamino, Di-($C_1$-$C_6$-Dialkyl)amino und Nitro ausgewählt sind, wie Phenyl, Methylphenyl (Tolyl), Dimethylphenyl (Xylyl), wie z. B. 2,6-Dimethylphenyl, Trimethylphenyl, wie z. B. 2,4,6-Trimethylphenyl, Ethylphenyl, Diethylphenyl, iso-Propylphenyl, tert.-Butylphenyl, Dodecylphenyl, Chlorphenyl, Dichlorphenyl, Trichlorphenyl, Fluorphenyl, Difluorphenyl, Trifluorphenyl, Tetrafluorphenyl, Pentafluorphenyl, 2,6-Dichlorphenyl, 4-Bromphenyl, Methoxyphenyl, Dimethoxyphenyl, Ethoxyphenyl, Hexyloxyphenyl, 2,6-Dimethoxyphenyl, 2-Nitrophenyl, 4-Nitrophenyl, 2,4-Dinitrophenyl, 2,6-Dinitrophenyl, 4-Dimethylaminophenyl, 4-Acetylphenyl, Methoxyethylphenyl, Ethoxymethylphenyl, Methylthiophenyl, Isopropylthiophenyl, tert.-Butylthiophenyl, $\alpha$-Naphthyl, $\beta$-Naphthyl, Methylnaphthyl, Isopropylnaphthyl, Chlornaphthyl, Ethoxynaphthyl, oder partiell fluoriertes Phenyl oder perfluoriertes Phenyl.

Wenn $R^1$ bis $R^9$ in den Verbindungen der Formel IV.a bis IV.v für $C_5$-$C_{12}$-Cycloalkyl stehen, so stehen $R^1$ bis $R^9$ vorzugsweise für unsubstituiertes Cycloalkyl, wie Cyclopentyl oder Cyclohexyl;
$C_5$-$C_{12}$-Cycloalkyl, das ein- oder zweifach substituiert ist, wobei die Substituenten unabhängig voneinander unter $C_1$-$C_6$-Alkyl, $C_1$-$C_6$-Alkoxy, $C_1$-$C_6$-Alkylsulfanyl oder Chlor ausgewählt sind, z. B. Butylcyclohexyl, Methoxycyclohexyl, Dimethoxycyclohexyl, Diethoxycyclohexyl, Butylthiocyclohexyl, Chlorcydohexyl, Dichlorcyclohexyl, Dichlorcyclopentyl;
$C_5$-$C_{12}$-Cycloalkyl, das ganz oder vollständig fluoriert ist.

Wenn $R^1$ bis $R^9$ in den Verbindungen der Formel IV.a bis IV.v für Polycyclyl stehen, so stehen $R^1$ bis $R^9$ vorzugsweise für $C_5$-$C_{12}$-Bicycloalkyl wie Norbornyl oder $C_5$-$C_{12}$-Bicydoalkenyl, wie Norbornenyl.

Wenn $R^1$ bis $R^9$ in den Verbindungen der Formel IV.a bis IV.v für $C_5$-$C_{12}$-Cycloalkenyl stehen, so stehen $R^1$ bis $R^9$ vorzugsweise für unsubstituiertes Cycloalkenyl, wie Cyclopent-2-en-1-yl, Cyclopent-3-en-1-yl, Cyclohex-2-en-1-yl, Cyclohex-1-en-1-yl, Cyclohexa-2,5-dien-1-yl oder partiell oder vollständig fluoriertes Cycloalkenyl.

Wenn $R^1$ bis $R^9$ in den Verbindungen der Formel IV.a bis IV.v für Heterocycloalkyl mit 5 oder 6 Ringatomen stehen, so stehen $R^1$ bis $R^9$ vorzugsweise für 1,3-Dioxolan-2-yl, 1,3-Dioxan-2-yl, 2-Methyl-1,3-dioxolan-2-yl, 4-Methyl-1,3-dioxolan-2-yl.

Wenn $R^1$ bis $R^9$ in den Verbindungen der Formel IV.a bis IV.v für Heteroaryl stehen, so stehen $R^1$ bis $R^9$ vorzugsweise für Furyl, Thienyl, Pyrryl, Pyridyl, Indolyl, Benzoxazolyl, Benzimidazolyl, Benzthiazolyl. Im Falle einer Substitution trägt Hetaryl 1, 2 oder 3 Substituenten, die unabhängig voneinander ausgewählt sind unter $C_1$-$C_6$-Alkyl, $C_1$-$C_6$-Alkoxy und Halogen, beispielsweise Dimethylpyridyl, Methylchinolyl, Dimethylpyrryl, Methoxyfuryl, Dimethoxypyridyl oder Difluorpyridyl.

Besonders bevorzugt stehen die Reste $R^1$ bis $R^9$ in den Verbindungen der Formel IV.a bis IV.v unabhängig voneinander für Wasserstoff,
unverzweigtes oder verzweigtes, unsubstituiertes oder ein bis mehrfach mit Hydroxy, Halogen, Phenyl, Cyano, $C_1$-$C_6$-Alkoxycarbonyl und/oder Sulfogruppe substituiertes $C_1$-$C_{18}$-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl, 1-Octadecyl, 2-Hydroxyethyl, Benzyl, 3-Phenyl-propyl, 2-Cyanoethyl, Methoxycarbonylmethyl, Ethoxycarbonylmethyl, n-Butoxycarbonylmethyl, tert-Butoxycarbonylmethyl, 2-(Methoxycarbonyl)-ethyl, 2-(Ethoxycarbonyl)-ethyl, 2-(n-Butoxy-carbonyl)-ethyl, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl oder 6-Hydroxyhexyl und 3-Sulfopropyl,
Hydroxyethyloxyalkyl, Reste von Oligo- und Polyalkylenglycolen, wie Polyethylenglycole und Polypropylenglycolen und deren Oligomere mit 2 bis 100 Einheiten und einem H oder einem $C_1$-$C_8$-Alkyl als Endgruppe, wie beispielsweise $R^AO$-$(CHR^B$-$CH_2$-$O)_n$-$CHR^B$-$CH_2$- oder $R^AO$-$(CH_2CH_2CH_2CH_2O)_n$-$CH_2CH_2CH_2CH_2O$-, worin $R^A$ und $R^B$ bevorzugt für H, Methyl oder Ethyl und n bevorzugt für 0 bis 3 steht, insbesondere 3-Oxabutyl, 3-Oxapentyl, 3,6-Dioxaheptyl, 3,6-Dioxaoctyl, 3,6,9-Trioxadecyl, 3,6,9-Trioxaundecyl, 3,6,9,12-Tetraoxatridecyl und 3,6,9,12-Tetraoxatetradecyl;
$C_2$-$C_4$-Alkenyl, wie Vinyl und Allyl; und
N,N-Di-$C_1$-$C_6$-alkylamino, wie beispielsweise N,N-Dimethylamino und N,N-Diethylamino.

Ganz besonders bevorzugt stehen die Reste $R^1$ bis $R^9$ unabhängig voneinander für Wasserstoff; $C_1$-$C_{18}$-Alkyl, wie Methyl, Ethyl, 1-Butyl, 1-Pentyl, 1-Hexyl, 1-Heptyl, 1-Octyl; Phenyl; 2-Hydroxyethyl; 2-Cyanoethyl; 2-(Alkoxycarbonyl)ethyl, wie 2-(Methoxycarbonyl)ethyl, 2-(Ethoxycarbonyl)ethyl oder 2-(n-Butoxycarbonyl)ethyl; N,N-($C_1$-$C_4$-Dialkyl)amino, wie N,N-Dimethylamino oder N,N-Diethylamino; Chlor sowie für Reste von Oligoalkylenglycol, wie $CH_3O$-$(CH_2CH_2O)_n$-$CH_2CH_2$- oder $CH_3CH_2O$-$(CH_2CH_2O)_n$-$CH_2CH_2$-, worin n für 0 bis 3 steht.

Bevorzugte Pyridiniumionen sind Verbindungen der Formel IV.a, worin einer der Reste $R^1$ bis $R^5$ für Methyl, Ethyl oder Chlor steht und die verbleibenden Reste $R^1$ bis $R^5$ für H stehen.

Weitere bevorzugte Pyridiniumionen sind Verbindungen der Formel IV.a, worin $R^3$ für Dimethylamino steht und die verbleibenden Reste $R^1$, $R^2$, $R^4$ und $R^5$ für H stehen. Weitere bevorzugte Pyridiniumionen sind Verbindungen der Formel IV.a, worin die Reste $R^1$ bis $R^5$ für H stehen.

Weitere bevorzugte Pyridiniumionen sind Verbindungen der Formel IV.a, worin $R^2$ für Carboxy oder Carboxamid steht und die verbleibenden Reste $R^1$, $R^2$, $R^4$ und $R^5$ für H stehen.

Weitere bevorzugte Pyridiniumionen sind Verbindungen der Formel IV.a, worin $R^1$ und $R^2$ oder $R^2$ und $R^3$ gemeinsam für 1,4-Buta-1,3-dienylen stehen und die verbleibenden Reste $R^1$, $R^2$, $R^4$ und $R^5$ für H stehen.

Besonders bevorzugte Pyridiniumionen sind Pyridinium, 2-Methylpyridinium, 2-Ethylpyridinium, 5-Ethyl-2-methylpyridinium und 2-Methyl-3-ethylpyridinium sowie 1-Methylpyridinium, 1-Ethylpyridinium, 1-(1-Butyl)pyridinium, 1-(1-Hexyl)pyridinium, 1-(1-Octyl)-pyridinium, 1-(1-Hexyl)-pyridinium, 1-(1-Octyl)-pyridinium, 1-(1-Dodecyl)-pyridinium, 1-(1-Tetradecyl)-pyridinium, t-(1-Hexadecyl)-pyridinium, 1,2-Dimethylpyridinium, 1-Ethyl-2-methylpyridinium, 1-(1-Butyl)-2-methylpyridinium, 1-(1-Hexyl)-2-methylpyridinium, 1-(1-Octyl)-2-methylpyridinium, 1-(1-Dodecyl)-2-methylpyridinium, 1-(1-Tetradecyl)-2-methylpyridinium, 1-(1-Hexadecyl)-2-methylpyridinium, 1-Methyl-2-ethylpyridinium, 1,2-Diethylpyridinium, 1-(1-Butyl)-2-ethylpyridinium, 1-(1-Hexyl)-2-ethylpyridinium, 1-(1-Octyl)-2-ethylpyridinium, 1-(1-Dodecyl)-2-ethylpyridinium, 9-(1-Tetradecyl)-2-ethylpyridinium, 1-(1-Hexadecyl)-2-ethylpyridinium, 1,2-Dimethyl-5-ethyl-pyridinium, 1,5-Diethyl-2-methyl-pyridinium, 1-(1-Butyl)-2-methyl-3-ethyl-pyridinium, 1-(1-Hexyl)-2-methyl-3-ethyl-pyridinium und 1-(1-Octyl)-2-methyl-3-ethyl-pyridinium, 1-(1-Dodecyl)-2-methyl-3-ethyl-pyridinium, 1-(1-Tetradecyl)-2-methyl-3-ethyl-pyridinium und 1-(1-Hexadecyl)-2-methyl-3-ethyl-pyridinium.

Bevorzugte Pyridaziniumionen sind Verbindungen der Formel IV.b, worin die Reste $R^1$ bis $R^4$ für H stehen, oder worin einer der Reste $R^1$ bis $R^4$ für Methyl oder Ethyl steht und die verbleibenden Reste $R^1$ bis $R^4$ für H stehen.

Bevorzugt Pyrimidiniumionen sind Verbindungen der Formel IV.c, worin $R^1$ für H, Methyl oder Ethyl steht und $R^2$ bis $R^4$ unabhängig voneinander für H oder Methyl stehen, oder worin $R^1$ für H, Methyl oder Ethyl steht, $R^2$ und $R^4$ für Methyl stehen und $R^3$ für H steht.

Bevorzugte Pyraziniumionen sind Verbindungen der Formel IV.d, worin $R^1$ für H, Methyl oder Ethyl steht und $R^2$ bis $R^4$ unabhängig voneinander für H oder Methyl stehen, oder worin $R^1$ für H, Methyl oder Ethyl steht und $R^2$ und $R^4$ für Methyl stehen und $R^3$ für H steht, oder worin $R^1$ bis $R^4$ für Methyl stehen oder worin $R^1$ bis $R^4$ für H stehen. Bevorzugte Imidazoliumionen sind Verbindungen der Formel IV.e, worin $R^1$ für H, Methyl, Ethyl, 1-Propyl, 1-Butyl, 1-Pentyl, 1-Hexyl, 1-Octyl, 2-Hydroxyethyl oder 2-Cyanoethyl steht und $R^2$ bis $R^4$ unabhängig voneinander für H, Methyl oder Ethyl stehen.

Besonders bevorzugte Imidazoliumionen der Formel IV.e sind 1-Methylimidazolium, 1-Ethylimidazolium, 1-(1-Propyl)-imidazolium, 1-(1-Allyl)-imidazolium, 1-(1-Butyl)-imidazolium, 1-(1-Octyl)-imidazolium, 1-(1-Dodecyl)-imidazolium, 1-(1-Tetradecyl)-imidazolium, 1-(1-Hexadecyl)-imidazolium, 1,3-Dimethylimidazolium, 1,3-Diethylimidazolium, 1-Ethyl-3-methylimidazolium, 1-(1-Butyl)-3-methylimidazolium, 1-(1-Butyl)-3-ethylimidazolium, 1-(1-Hexyl)-3-methyl-imidazolium, 1-(1-Hexyl)-3-ethylimidazolium, 1-(1-Hexyl)-3-butyl-imidazolium, 1-(1-Octyl)-3-methylimidazolium, 1-(1-Octyl)-3-ethylimidazolium, 1-(1-Octyl)-3-butylimidazolium, 1-(1-Dodecyl)-3-methylimidazolium, 1-(1-Dodecyl)-3-ethylimidazolium, 1-(1-Dodecyl)-3-butylimidazolium, 1-(1-Dodecyl)-3-octylimidazolium, 1-(1-Tetradecyl)-3-methylimidazolium, 1-(1-Tetradecyl)-3-ethylimidazolium, 1-(1-Tetradecyl)-3-butylimidazolium, 1-(1-Tetradecyl)-3-octylimidazolium, 1-(1-Hexadecyl)-3-methylimidazolium, 1-(1-Hexadecyl)-3-ethylimidazolium, 1-(1-Hexadecyl)-3-butylimidazolium, 1-(1-Hexadecyl)-3-octylimidazolium, 1,2-Dimethylimidazolium, 1,2,3-Trimethylimidazolium, 1-Ethyl-2,3-dimethylimidazolium, 1-(1-Butyl)-2,3-dimethylimidazolium, 1-(1-Hexyl)-2,3-dimethyl-imidazolium, 1-(1-Octyl)-2,3-dimethylimidazolium, 1,4-Dimethylimidazolium, 1,3,4-Trimethylimidazolium, 1,4-Dimethyl-3-ethylimidazolium, 3-Methylimidazolium, 3-Ethylimidazolium, 3-n-Propylimidazolium, 3-n-Butylimidazolium, 1,4-Dimethyl-3-octylimidazolium, 1,4,5-Trimethylimidazolium, 1,3,4,5-Tetramethylimidazolium, 1,4,5-Trimethyl-3-ethylimidazolium, 1,4,5-Trimethyl-3-butylimidazolium, 1,4,5-Trimethyl-3-octylimidazolium, 1-Prop-1-en-3-yl-3-methylimidazolium und 1-Prop-1-en-3-yl-3-butylimidazolium.

Bevorzugte Pyrazoliumionen sind Verbindungen der Formeln IV.f, IV.g bzw. IV.g', worin $R^1$ für H, Methyl oder Ethyl steht und $R^2$ bis $R^4$ unabhängig voneinander für H oder Methyl stehen.

Weitere bevorzugte Pyrazoliumionen sind Verbindungen der Formel IV.h, worin $R^1$ bis $R^4$ unabhängig voneinander für H oder Methyl stehen.

Besonders bevorzugte Pyrazoliumionen sind 1,4-Dimethylpyrazolium und 1,2,4-Trimethylpyrazolium genannt.

Bevorzugte 1-Pyrazoliniumionen sind Verbindungen der Formel IV.i, worin $R^1$ bis $R^6$ unabhängig voneinander für H oder Methyl stehen.

Bevorzugte 2-Pyrazoliniumionen sind Verbindungen der Formel IV.j bzw. IV.j', worin $R^1$ für H, Methyl, Ethyl oder Phenyl steht und $R^2$ bis $R^6$ unabhängig voneinander für H oder Methyl stehen.

Bevorzugte 3-Pyrazoliniumionen sind Verbindungen der Formel IV.k. bzw..IV.k', worin $R^1$ und $R^2$ unabhängig voneinander für H, Methyl, Ethyl oder Phenyl stehen und $R^3$ bis $R^6$ unabhängig voneinander für H oder Methyl stehen.

Bevorzugte Imidazoliniumionen sind Verbindungen der Formel (IV.l), worin $R^1$ und $R^2$ unabhängig voneinander

für H, Methyl, Ethyl, 1-Butyl oder Phenyl stehen, $R^3$ und $R^4$ unabhängig voneinander für H, Methyl oder Ethyl stehen und $R^5$ und $R^6$ unabhängig voneinander für H oder Methyl stehen.

Weitere bevorzugte Imidazoliniumionen sind Verbindungen der Formel IV.m bzw. IV.m', worin $R^1$ und $R^2$ unabhängig voneinander für H, Methyl oder Ethyl stehen und $R^3$ bis $R^6$ unabhängig voneinander für H oder Methyl stehen.

Weitere bevorzugte Imidazoliniumionen sind Verbindungen der Formel IV.n bzw. IV.n', worin $R^1$ bis $R^3$ unabhängig voneinander für H, Methyl oder Ethyl stehen und $R^4$ bis $R^6$ unabhängig voneinander für H oder Methyl stehen.

Bevorzugte Thiazoliumionen sind verbindungen der Formel IV.o bzw. IV.o', worin $R^1$ für H, Methyl, Ethyl oder Phenyl steht und $R^2$ und $R^3$ unabhängig voneinander für H oder Methyl stehen.

Bevorzugte Oxazoliumionen sind Verbindungen der Formel IV.p, worin $R^1$ für H, Methyl, Ethyl oder Phenyl steht und $R^2$ und $R^3$ unabhängig voneinander für H oder Methyl stehen.

Bevorzugte 1,2,4-Triazoliumionen sind Verbindungen der Formeln IV.q, IV.q' bzw. IV.q", worin $R^1$ und $R^2$ unabhängig voneinander für H, Methyl, Ethyl oder Phenyl stehen und $R^3$ für H, Methyl oder Phenyl steht.

Bevorzugte 1,2,3-Triazoliumionen sind Verbindungen der Formeln IV.r, IV.r' bzw. IV.r", worin $R^1$ für H, Methyl oder Ethyl steht, $R^2$ und $R^3$ unabhängig voneinander für H oder Methyl stehen, oder $R^2$ und $R^3$ zusammen für 1,4-Buta-1,3-dienylen stehen.

Bevorzugte Pyrrolidiniumionen sind Verbindungen der Formel IV.s, worin $R^1$ für H, Methyl, Ethyl oder Phenyl steht und $R^2$ bis $R^9$ unabhängig voneinander für H oder Methyl stehen.

Bevorzugte Imidazolidiniumionen sind Verbindungen der Formel IV.t, worin $R^1$ und $R^4$ unabhängig voneinander für H, Methyl, Ethyl oder Phenyl stehen und $R^2$, $R^3$ und $R^5$ bis $R^8$ unabhängig für H oder Methyl stehen.

Bevorzugt Diazabicycloalkeniumionen der Formeln IV.u und IV.v sind ausgewählt unter kationischen Derivaten von 1,5-Diazabicyclo[4.3.0]non-5-en (DBN) und 1,8-Diazabicyclo[5.4.0]-undec-7-en (DBU).

In einer besonders bevorzugten Ausführungsform des erfindungsgemäßen Verfahrens wird eine ionische Flüssigkeit IL aufgearbeitet, die wenigstens ein Kation aufweist, das ausgewählt ist unter den zuvor genannten Imidazoliumionen und den zuvor genannten Pyrazoliumionen. Ganz besonders bevorzugt ist das Kation der ionischen Flüssigkeit unter den zuvor genannten Imidazoliumionen ausgewählt. Bezüglich bevorzugter Imidazoliumionen und Pyrazoliumionen wird auf die zuvor gemachten Ausführungen in vollem Umfang Bezug genommen.

Die vorliegende Erfindung wird im Folgenden anhand von Beispielen näher erläutert.

Beispiele

Zur Bestimmung der im Folgenden angegebenen pH-Werte ionischer Flüssigkeiten wurde jeweils 1 ml der ionischen Flüssigkeit mit Wasser auf ein Gesamtvolumen von 10 ml verdünnt und der pH-Wert des so erhaltenen Gemisches durch eine Glaselektrode bestimmt.

Beispiel 1: Aufarbeitung von saurem EMIM-Tosylat

a) Neutralisation von EMIM-Tosylat

1-Ethyl-3-methylimidazoliumtosylat (EMIM-Tosylat) wurde durch Reaktion von EMIM-Chlorid mit p-Toluolsulfonsäure bereitgestellt. Das so erhaltene EMIM-Tosylat wies einen pH-Wert von 2,5 auf (bestimmt gemäß obiger Methode). Zu diesem EMIM-Tosylat (pH = 2,5; 50,3 g) wurde eine Lösung von EMIM-Butoxylat (1 M in Methanol; 5,186 g) gegeben. Das erhaltene Gemisch wies einen pH-Wert von 6,9 auf (bestimmt gemäß obiger Methode). Methanol und das bei der Neutralisation entstandene Butanol wurden am Rotationsverdampfer unter vermindertem Druck und bei 120 °C entfernt. Der Rückstand wies einen pH-Wert von 6,7 auf (bestimmt gemäß obiger Methode).

b) Überprüfung der Eigenschaften von EMIM-Tosylat (pH = 6,9)

Ein Gemisch aus Trimethylborat (0,642 g), Methanol (5,774 g) und EMIM-Tosylat aus Beispiel 1.a) (pH = 6,9; 57,739 g) wurde in einem Edelstahltiegel (1,457 l) vorgelegt und einer Druckwärmestaumessung unterzogen. Bei der Druckwännestauprüfung handelt es sich um einen adiabatischen Durchgehversuch in einem Reaktionsgefäß mit Druck- und Temperaturnachführung. Mit Stickstoff wurde ein Anfangsdruck von 2 bar (absolut) eingestellt. Die Innentemperatur des Edelstahltiegels wurde für 24 Stunden auf 130 °C und für weitere 59 Stunden auf 170 °C erwärmt. Eine Druckerhöhung fand während dieser Zeit nicht statt. Durch Headspace-GC-MS-Analytik konnte kein Dimethylether als Zersetzungsprodukt nachgewiesen werden. Das Gemisch enthaltend die aufgearbeitete ionische Flüssigkeit ist thermisch stabil.

3. Überprüfung der Eigenschaften von EMIM-Tosylat (pH = 2,5) (Vergleichsbeispiel)

Eine Gemisch aus Trimethylborat (0,327 g), Methanol (2,941 g) und EMIM-Tosylat (pH = 2,5; 29,412 g) wurde

in einem Edelstahltiegel (1,457 l) vorgelegt und einer Druckwärmestaumessung unterzogen. Mit Stickstoff wurde ein Anfangsdruck von 2 bar (absolut) eingestellt. Der Edelstahltiegel wurde auf 130 °C Innentemperatur erwärmt. Der Druck stieg innerhalb der ersten Stunde auf etwa 10 bar und innerhalb von 40 Stunden auf über 50 bar. Durch Headspace-GC-MS-Analytik konnte Dimethylether als Zersetzungsprodukt nachgewiesen werden.

**Patentansprüche**

1. Verfahren zur Aufarbeitung einer ionischen Flüssigkeit (IL), ausgewählt unter Verbindungen der allgemeinen Formel (I),

$$[A]^{+}\ (1/n)^{*}[Y]^{n-} \qquad (I)$$

worin $[A]^+$ für ein quartäres Ammonium-Kation und $(1/n)^*[Y]^{n-}$ für ein Anionäquivalent eines n-fach geladenen Anions steht,
wobei die ionische Flüssigkeit (IL) einen sauren oder basischen pH-Wert aufweist,
bei dem man der ionischen Flüssigkeit (IL) ein konjugiertes Säure-Base-Paar, ausgewählt unter Verbindungen der Formel (II) zusetzt,

$$[A]^{+}\ [X]^{-} \qquad (II)$$

worin $[A]^+$ die für die ionische Flüssigkeit gegebene Bedeutung aufweist und [X]-für eine konjugierte Base steht.

2. Verfahren gemäß Anspruch 1, wobei die ionische Flüssigkeit (IL) einen pH-Wert im Bereich von 1 bis 6,9 aufweist, bei dem das konjugiertes Säure-Base-Paar der Formel (II) ausgewählt ist unter basischen Verbindungen.

3. Verfahren gemäß Anspruch 2, wobei die konjugierte Base $[X]^-$ für ein Anion der Formel $-OR^1$ steht, worin $R^1$ für Alkyl oder Cycloalkyl steht.

4. Verfahren gemäß Anspruch 3, worin $R^1$ für $C_1$-$C_4$-Alkyl steht.

5. Verfahren nach einem der vorhergehenden Ansprüche, bei dem man das Neutralisationsprodukt der Formel X-H aus der ionischen Flüssigkeit (IL) abtrennt.

6. Verfahren nach Anspruch 5, bei dem man die Abtrennung des Neutralisationsproduktes der Formel X-H durch Destillation vornimmt.

7. Verfahren gemäß einem der Ansprüche 2 bis 6, wobei $[Y]^{n-}$ in den Verbindungen der Formel (I) ausgewählt ist unter Verbindungen der Formeln $(R^a)SOs$-, $(R^a)SO_2$-, $(R^a)PO_3^{2-}$, $(R^a)(R^b)PO_2^-$, $(R^a)PO_2^{2-}$, $(R^a)(R^b)PO^-$ und $(R^a)PO^{2-}$, worin $R^a$ und $R^b$ unabhängig voneinander für OH, Alkyl, Alkoxy, Aryl, Aryloxy, Cycloalkyl, Cycloalkoxy, Heterocycloalkyl, Heterocycloalkyloxy, Heteroaryl oder Heteroaryloxy stehen.

8. Verfahren gemäß Anspruch 7, wobei $[Y]^{n-}$ ausgewählt ist unter Verbindungen der Formeln $(R^a)SO_3^-$ und $(R^a)(R^b)PO_2^-$, worin $R^a$ und $R^b$ unabhängig voneinander für Alkyl, Alkoxy, Aryl oder Aryloxy stehen.

9. Verfahren gemäß Anspruch 8, wobei $[Y]^{n-}$ für eine Verbindung der Formel $(R^a)SO_3^-$ steht.

10. Verfahren gemäß einem der vorhergehenden Ansprüche, wobei $[A]^+$ in den Verbindungen der Formel (I) und (II) ausgewählt ist unter Verbindungen, die eine molare Masse von weniger als 1000 g/mol aufweisen.

11. Verfahren gemäß einem der vorhergehenden Ansprüche, wobei $[A]^+$ für ein gegebenenfalls substituiertes Imidazoliumion steht, das ausgewählt ist unter 1-Methylimidazolium, 1-Ethylimidazolium, 1-(1-Propyl)-imidazolium, 1-(1-Allyl)-imidazolium, 1-(1-Butyl)-imidazolium, 1-(1-Octyl)-imidazolium, 1-(1-Dodecyl)-imidazolium, 1-(1-Tetradecyl)-imidazolium, 1-(1-Hexadecyl)-imidazolium, 1,3-Dimethylimidazolium, 1,3-Diethylimidazolium, 1-Ethyl-3-methylimidazolium, 1-(1-Butyl)-3-methylimidazolium, 1-(1-Butyl)-3-ethylimidazolium, 1-(1-Hexyl)-3-methyl-imidazolium, 1-(1-Hexyl)-3-ethylimidazolium, 1-(1-Hexyl)-3-butyl-imidazolium, 1-(1-Octyl)-3-methylimidazolium, 1-(1-Octyl)-3-ethylimidazolium, 1-(1-Octyl)-3-butylimidazolium, 1-(1-Dodecyl)-3-methylimidazolium, 1-(1-Dodecyl)-3-ethylimidazolium, 1-(1-Dodecyl)-3-butylimidazolium, 1-(1-Dodecyl)-3-octylimidazolium, 1-(1-Tetradecyl)-3-methylimidazolium, 1-(1-Tetradecyl)-3-ethylimidazolium, 1-(1-Tetradecyl)-3-butylimidazolium, 1-(1-Tetradecyl)-3-octylimidazolium, 1-(1-Hexadecyl)-3-methylimidazolium, 1-(1-Hexadecyl)-3-ethylimidazolium, 1-(1-Hexadecyl)-3-butylimidazolium, 1-(1-Hexadecyl)-3-octylimidazolium, 1,2-Dimethylimidazolium, 1,2,3-Trimethylimidazolium, 1-Ethyl-2,3-dimethylimidazolium, 1-(1-Butyl)-2,3-dimethylimidazolium, 1-(1-Hexyl)-2,3-dimethyl-imidazolium, 1-(1-Octyl)-2,3-dimethylimidazolium, 1,4-Dimethylimidazolium, 1,3,4-Trimethylimidazolium, 1,4-Dimethyl-3-ethylimidazolium, 3-Methylimidazolium, 3-Ethylimidazolium, 3-n-Propylimidazolium, 3-n-Butylimidazolium, 1,4-Dimethyl-3-octylimidazolium, 1,4,5-Trimethylimidazolium, 1,3,4,5-Tetramethylimidazolium, 1,4,5-Trimethyl-3-ethylimidazolium, 1,4,5-Trimethyl-3-butylimidazolium, 1,4,5-Trimethyl-3-octylimidazolium, 1-Prop-1-en-3-yl-3-methylimidazolium und 1-Prop-1-en-3-yl-3-butylimidazolium.

## Claims

1. A process for the work-up of an ionic liquid (IL) selected from among compounds of the general formula (I),

$$[A]^+ \ (1/n)*[Y]^{n-} \qquad (I)$$

where $[A]^+$ is a quaternary ammonium cation and $(1/n)*[Y]^{n-}$ is one anion equivalent of an n-fold charged anion, where the ionic liquid (IL) has an acidic or basic pH,
which comprises adding a conjugate acid-base pair selected from among compounds of the formula (II),

$$[A]^+ \ [X]^- \qquad (II)$$

where $[A]^+$ has the meaning given for the ionic liquid and $[X]^-$ is a conjugate base,
to the ionic liquid (IL).

2. The process according to claim 1, wherein the ionic liquid (IL) has a pH in the range from 1 to 6.9 and the conjugate acid-base pair of the formula (II) is selected from among basic compounds.

3. The process according to claim 2, wherein the conjugate base $[X]^-$ is an anion of the formula $^-OR^1$, where $R^1$ is alkyl or cycloalkyl.

4. The process according to claim 3, wherein $R^1$ is $C_1$-$C_4$-alkyl.

5. The process according to any of the preceding claims, wherein the neutralization product of the formula X-H is separated off from the ionic liquid (IL).

6. The process according to claim 5, wherein the neutralization product of the formula X-H is separated off by distillation.

7. The process according to any of claims 2 to 6, wherein $[Y]^{n-}$ in the compounds of the formula (I) is selected from among compounds of the formulae $(R^a) SO_3^-$, $(R^a) SO_2^-$, $(R^a)PO_3^{2-}$, $(R^a) (R^b)PO_2^-$, $(R^a)PO_2^{2-}$, $(R^a) (R^b)PO^-$ and $(R^a)PO^{2-}$,
where $R^a$ and $R^b$ are each, independently of one another, OH, alkyl, alkoxy, aryl, aryloxy, cycloalkyl, cycloalkoxy, heterocycloalkyl, heterocycloalkyloxy, heteroaryl or heteroaryloxy.

8. The process according to claim 7, wherein $[Y]^{n-}$ is selected from among compounds of the formulae $(R^a)SO_3^-$ and $(R^a)(R^b)PO_2^-$, where $R^a$ and $R^b$ are each, independently of one another, alkyl, alkoxy, aryl or aryloxy.

9. The process according to claim 8, wherein $[Y]^{n-}$ is a compound of the formula $(R^a)SO_3^-$.

10. The process according to any of the preceding claims, wherein $[A]^+$ in the compounds of the formulae (I) and (II) is selected from among compounds having a molar mass of less than 1000 g/mol.

11. The process according to any of the preceding claims, wherein $[A]^+$ is an optionally substituted imidazolium ion which is selected from among 1-methylimidazolium, 1-ethylimidazolium, 1-(1-propyl)imidazolium, 1-(1-allyl)imidazolium, 1-(1-butyl)imidazolium, 1-(1-octyl)imidazolium, 1-(1-dodecyl)imidazolium, 1-(1-tetradecyl)-imidazolium, 1-(1-hexadecyl)imidazolium, 1,3-dimethylimidazolium, 1,3-diethylimidazolium, 1-ethyl-3-methylimidazolium, 1-(1-butyl)-3-methylimidazolium, 1-(1-butyl)-3-ethylimidazolium, 1-(1-hexyl)-3-methylimidazolium, 1-(1-hexyl)-3-ethylimidazolium, 1-(1-hexyl)-3-butylimidazolium, 1-(1-octyl)-3-methylimidazolium, 1-(1-octyl)-3-ethylimidazolium, 1-(1-octyl)-3-butylimidazolium, 1-(1-dodecyl)-3-methylimidazolium, 1-(1-dodecyl)-3-ethylimidazolium, 1-(1-dodecyl)-3-butylimidazolium, 1-(1-dodecyl)-3-octylimidazolium, 1-(1-tetradecyl)-3-methylimidazolium, 1-(1-tetradecyl)-3-ethylimidazolium, 1-(1-tetradecyl)-3-butylimidazolium, 1-(1-tetradecyl)-3-octylimidazolium, 1-(1-hexadecyl)-3-methylimidazolium, 1-(1-hexadecyl)-3-ethylimidazolium, 1-(1-hexadecyl)-3-butylimidazolium, 1-(1-hexadecyl)-3-octylimidazolium, 1,2-dimethylimidazolium, 1,2,3-trimethylimidazolium, 1-ethyl-2,3-dimethylimidazolium, 1-(1-butyl)-2,3-dimethylimidazolium, 1-(1-hexyl)-2,3-dimethylimidazolium, 1-(1-octyl)-2,3-dimethylimidazolium, 1,4-dimethylimidazolium, 1,3,4-trimethylimidazolium, 1,4-dimethyl-3-ethylimidazolium, 3-methylimidazolium, 3-ethylimidazolium, 3-n-propylimidazolium, 3-n-butylimidazolium, 1,4-dimethyl-3-octylimidazolium, 1,4,5-trimethylimidazolium, 1,3,4,5-tetramethylimidazolium, 1,4,5-trimethyl-3-ethylimidazolium, 1,4,5-trimethyl-3-butylimidazolium, 1,4,5-trimethyl-3-octylimidazolium, 1-prop-l-en-3-yl-3-methylimidazolium and 1-prop-1-en-3-yl-3-butylimidazolium.

## Revendications

1. Procédé de traitement d'un liquide ionique (IL), choisi parmi les composés de formule générale (I)

$$[A]^+ \quad (1/n)*[Y]^{n-} \qquad\qquad (I)$$

dans laquelle $[A]^+$ représente un cation ammonium quaternaire et $(1/n)*[Y]^{n-}$ représente un équivalent anionique d'un anion chargé n fois,
le liquide ionique (IL) présentant un pH acide ou basique,
dans lequel une paire acide-base conjuguée choisie parmi les composés de formule (II) est ajoutée au liquide ionique (IL)

$$[A]^+ \ [X]^- \quad (II)$$

dans laquelle $[A]^+$ a la signification indiquée pour le liquide ionique et $[X]^-$ représente une base conjuguée.

2. Procédé selon la revendication 1, le liquide ionique (IL) présentant un pH dans la plage allant de 1 à 6,9, dans lequel la paire acide-base conjuguée de formule (II) est choisie parmi les composés basiques.

3. Procédé selon la revendication 2, dans lequel la base conjuguée $[X]^-$ représente un anion de formule $-OR^1$, $R^1$ représentant alkyle ou cycloalkyle.

4. Procédé selon la revendication 3, dans lequel $R^1$ représente alkyle en $C_1$-$C_4$.

5. Procédé selon l'une quelconque des revendications précédentes, dans lequel le produit de neutralisation de formule X-H est séparé du liquide ionique (IL).

6. Procédé selon la revendication 5, dans lequel la séparation du produit de neutralisation de formule X-H est réalisée par distillation.

7. Procédé selon l'une quelconque des revendications 2 à 6, dans lequel $[Y]^{n-}$ dans les composés de formule (I) est choisi parmi les composés de formule $(R^a)\ SO_3^-$, $(R^a)\ SO_2^-$, $(R^a)PO_3^{2-}$, $(R^a)\ (R^b)PO_2^-$, $(R^a)PO_2^{2-}$, $(R^a)\ (R^b)PO^-$ et $(R^a)PO^{2-}$,
$R^a$ et $R^b$ représentant indépendamment l'un de l'autre OH, alkyle, alcoxy, aryle, aryloxy, cycloalkyle, cycloalcoxy, hétérocycloalkyle, hétérocycloalkyloxy, hétéroaryle ou hétéroaryloxy.

8. Procédé selon la revendication 7, dans lequel $[Y]^{n-}$ est choisi parmi les composés de formule $(R^a)SO_3^-$ et $(R^a)(R^b)PO_2^-$, $R^a$ et $R^b$ représentant indépendamment l'un de l'autre alkyle, alcoxy, aryle ou aryloxy.

9. Procédé selon la revendication 8, dans lequel $[Y]^{n-}$ représente un composé de formule $(R^a)SO_3^-$.

10. Procédé selon l'une quelconque des revendications précédentes, dans lequel $[A]^+$ dans les composés de formule (I) et (II) est choisi parmi les composés qui présentent une masse molaire inférieure à 1 000 g/mol.

11. Procédé selon l'une quelconque des revendications précédentes, dans lequel $[A]^+$ représente un ion imidazolium éventuellement substitué, qui est choisi parmi le 1-méthylimidazolium, le 1-éthylimidazolium, le 1-(1-propyl)-imidazolium, le 1-(1-allyl)-imidazolium, le 1-(1-butyl)-imidazolium, le 1-(1-octyl)-imidazolium, le 1-(1-dodécyl)-imidazolium, le 1-(1-tétradécyl)-imidazolium, le 1-(1-hexadécyl)-imidazolium, le 1,3-diméthylimidazolium, le 1,3-diéthylimidazolium, le 1-éthyl-3-méthylimidazolium, le 1-(1-butyl)-3-méthylimidazolium, le 1-(1-butyl)-3-éthylimidazolium, le 1-(1-hexyl)-3-méthylimidazolium, le 1-(1-hexyl)-3-éthylimidazolium, le 1-(1-hexyl)-3-butyl-imidazolium, le 1-(1-octyl)-3-méthylimidazolium, le 1-(1-octyl)-3-éthylimidazolium, le 1-(1-octyl)-3-butylimidazolium, le 1-(1-dodécyl)-3-méthylimidazolium, le 1-(1-dodécyl)-3-éthylimidazolium, le 1-(1-dodécyl)-3-butylimidazolium, le 1-(1-dodécyl)-3-octylimidazolium, le 1-(1-tétradécyl)-3-méthylimidazolium, le 1-(1-tétradécyl)-3-éthylimidazolium, le 1-(1-tétradécyl)-3-butylimidazolium, le 1-(1-tétradécyl)-3-octylimidazolium, le 1-(1-hexadécyl)-3-méthylimidazolium, le 1-(1-hexadécyl)-3-éthylimidazolium, le 1-(1-hexadécyl)-3-butylimidazolium, le 1-(1-hexadécyl)-3-octylimidazolium, le 1,2-diméthylimidazolium, le 1,2,3-triméthylimidazolium, le 1-éthyl-2,3-diméthylimidazolium, le 1-(1-butyl)-2,3-diméthylimidazolium, le 1-(1-hexyl)-2,3-diméthyl-imidazolium, le 1-(1-octyl)-2,3-diméthylimidazolium, le 1,4-diméthylimidazolium, le 1,3,4-triméthylimidazolium, le 1,4-diméthyl-3-éthylimidazolium, le 3-méthylimidazolium, le 3-éthylimidazolium, le 3-n-propylimidazolium, le 3-n-butylimidazolium, le 1,4-diméthyl-3-octylimidazolium, le 1,4,5-triméthylimidazolium, le 1,3,4,5-tétraméthylimidazolium, le 1,4,5-triméthyl-3-éthylimidazolium, le 1,4,5-triméthyl-3-butylimidazolium, le 1,4,5-triméthyl-3-octylimidazolium, le 1-prop-1-én-3-yl-3-méthylimidazolium et le 1-prop-1-én-3-yl-3-butylimidazolium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10202838 A1


### In der Beschreibung aufgeführte Nicht-Patentliteratur


- **K. N. MARSH et al.** *Fluid Phase Equilibria,* 2004, vol. 219, 93-98
- **J. G. HUDDLESTON et al.** *Green Chemistry,* 2001, vol. 3, 156-164
- *Angew. Chem.,* 2000, vol. 112, 3926-3945